# EUROPEAN PATENT SPECIFICATION

(11) **EP 1 656 360 B1**
(45) Date of publication and mention of the grant of the patent: **15.08.2007**
(21) Application number: 04755044.7
(22) Date of filing: 12.06.2004
(51) Int. Cl.: C07D 277/12, A61K 31/426, A61P 5/26

(54) **THIAZOLINE DERIVATIVES AS SELECTIVE ANDROGEN RECEPTOR MODULATORS (SARMS)**
THIAZOLINDERIVATE ALS SELEKTIVE ANDROGENREZEPTORMODULATOREN (SARMS)
DERIVES DE THIAZOLINE SERVANT DE MODULATEURS DE RECEPTEUR DES ANDROGENES SELECTIFS (SARMS)

(30) Priority: 13.06.2003 US 478712 P
(43) Date of publication of application: 17.05.2006
(73) Proprietor: JANSSEN PHARMACEUTICA N.V., 2340 Beerse (BE)
(72) Inventor: NG, Raymond A., c/o Johnson & Johnson Pharmaceut., Raritan, NJ 08869-0602 (US); SUI, Zhihua, c/o Johnson & Johnson Pharmaceutical, Raritan, NJ 08869-0602 (US)
(74) Representative: Mercer, Christopher Paul
(86) International application number: PCT/US2004/018665
(87) International publication number: WO 2004/113309

(56) References cited:
- WO-A-02/16310
- MATHUR K B ET AL: "STUDIES IN POTENTIAL ANTIMYCOBACTERIAL AGENTS: PART XVI - SYNTHESIS OF SOME 2-(2'-HYDROXYPHENYL)-2-THIAZOLINE-4-N-SUBS TITUTED CARBOXYAMIDES" JOURNAL OF SCIENTIFIC & INDUSTRIAL RESEARCH, XX, XX, vol. 21B, 1962, pages 34-37, XP008024535

## Description

### FIELD OF THE INVENTION

The present invention is directed to novel thiazoline derivatives, pharmaceutical compositions containing them and their use in the treatment of disorders and conditions modulated by the androgen receptor. More particularly, the compounds of the present invention are useful in the treatment of prostate carcinoma, benign prostatic hyperplasia (BPH), hirsitutism, alopecia, anorexia nervosa, breast cancer, acne, AIDS, cachexia, as a male contraceptive, and as a male performance enhancer.

### BACKGROUND OF THE INVENTION

Androgens are the anabolic steroid hormones of animals, controlling muscle and skeletal mass, the maturation of the reproductive system, the development of secondary sexual characteristics and the maintenance of fertility in the male. In women, testosterone is converted to estrogen in most target tissues, but androgens themselves may play a role in normal female physiology, for example, in the brain. The chief androgen found in serum is testosterone, and this is the effective compound in tissues such as the testes and pituitary. In prostate and skin, testosterone is converted to dihydrotestosterone (DHT) by the action of 5α-reductase. DHT is a more potent androgen than testosterone because it binds more strongly to the androgen receptor.

Like all steroid hormones, androgens bind to a specific receptor inside the cells of target tissues, in this case the androgen receptor. This is a member of the nuclear receptor transcription factor family. Binding of androgen to the receptor activates it and causes it to bind to DNA binding sites adjacent to target genes. From there it interacts with coactivator proteins and basic transcription factors to regulate the expression of the gene. Thus, via its receptor, androgens cause changes in gene expression in cells. These changes ultimately have consequences on the metabolic output, differentiation or proliferation of the celll that are visible in the physiology of the target tissue.

Although modulators of androgen receptor function have been employed clinically for some time, both the steroidal (Basaria, S., Wahlstrom, J.T., Dobs, A.S., J. Clin Endocrinol Metab (2001), 86, pp5108-5117; Shahidi, N.T., Clin Therapeutics, (2001), 23, pp1355-1390), and non-steroidal (Newling, D.W., Br. J. Urol., 1996, 77 (6), pp 776-784) compounds have significant liabilities related to their pharmacological parameters, including gynecomastia, breast tenderness and hepatoxicity. In addition, drug-drug interactions have been observed in patients receiving anticoagulation therapy using coumarins. Finally, patients with aniline sensitivities could be compromised by the metabolites of non-steroidal antiandrogens.

WO 02/16310 relates to alleged tissue-selective androgen receptor targeting agents which supposedly demonstrate androgenic and anabolic activity of a nonsteroidal ligand for the androgen receptor.

Non-steroidal agonists and antagonists of the androgen receptor are useful in the treatment of a variety of disorders and diseases. More particularly, agonists of the androgen receptor could be employed in the treatment of prostate cancer, benign prostatic hyperplasia, hirsutism in women, alopecia, anorexia nervosa, breast cancer and acne. Antagonists of the androgen receptor could be employed in male contraception, male performance enhancement, as well as In the treatment of cancer, AIDS, cachexia, and other disorders.

Nonetheless, there exists a need for small molecule, non-steroidal antagonists of the androgen receptor. We now describe a novel series of thiazoline derivatives as androgen receptor modulators.

### SUMMARY OF THE INVENTION

The present invention is directed to a compound of formula (I) 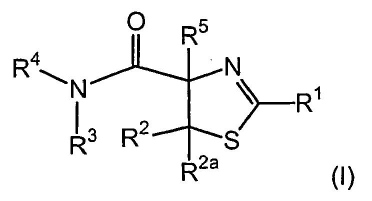 wherein
R¹ is selected from the group consisting of aryl and heteroaryl; wherein the heteroaryl group is bound through a carbon atom;
wherein the aryl or heteroaryl group is optionally substituted with one to four substituent independently selected from halogen, alkyl, halogen substituted alkyl, lower alkyl ester, cyano, N(R^{A})₂C(O)-, lower alkyl-C(O)-NR^{A}-, lower alkyl-S(O)₀₋₂-, phenyl-S(O)₀₋₂, lower alkyl-S(O)₀₋₂-NR^{A}-, phenyl-S(O)₀₋₂-NR^{A}- and trifluoromethyl-sulfonyl;
wherein the phenyl is optionally substituted with one or more substitutents independently selected from halogen, lower alkyl, lower alkoxy, hydroxy, carboxy, cyano, nitro, amino, lower alkylamino or di(lower alkyl)amino;
R^{A} is independently selected from hydrogen or lower alkyl;
R² and R^{2a} are each independently selected from the group consisting of hydrogen, lower alkyl and halogen substituted lower alkyl;
R³ is selected from the group consisting of hydrogen and lower alkyl;
R⁴ is selected from the group consisting of aryl and heteroaryl; wherein the heteroaryl group is bound through a carbon atom;
wherein the aryl or heteroaryl group is optionally substituted with one to four substituent independently selected from halogen, alkyl, halogen substituted alkyl, lower alkyl ester, cyano, N(R^{A})₂C(O)-, lower alkyl-C(O)-NR^{A}-, lower alkyl-S(O)₀₋₂-, phenyl-S(O)₀₋₂, lower alkyl-S(O)₀₋₂-NR^{A}-, phenyl-S(O)₀₋₂-NR^{A}- and trifluoromethyl-sulfonyl;
wherein the phenyl is optionally substituted with one or more substitutents independently selected from halogen, lower alkyl, lower alkoxy, hydroxy, carboxy, cyano, nitro, amino, lower alkylamino or di(lower alkyl)amino;
R⁵ is selected from the group consisting of hydrogen, lower alkyl and halogen substituted lower alkyl;
or a pharmaceutically acceptable salt thereof.

Illustrative of the invention is a pharmaceutical composition comprising a pharmaceutically acceptable carrier and any of the compounds described above. An illustration of the invention is a pharmaceutical composition made by mixing any of the compounds described above and a pharmaceutically acceptable carrier. Illustrating the invention is a process for making a pharmaceutical composition comprising mixing any of the compounds described above and a pharmaceutically acceptable carrier.

Exemplifying the invention are uses of the compounds of formula I for the manufacture of a medicament for treating disorders and conditions modulated by the androgen receptor in a subject in need thereof comprising administering to the subject a therapeutically effective amount of any of the compounds or pharmaceutical compositions described above.

An example of the invention is a use of the compounds of formula I for the manufacture of a medicament for treating an androgen treating an androgen receptor modulated disorder selected from the group consisting of prostate carcinoma, benign prostatic hyperplasia, hirsutism, or for male contraception, in a subject in need thereof comprising administering to the subject an effective amount of any of the compounds or pharmaceutical compositions described above.

Another example of the invention is the use of any of the compounds described herein in the preparation of a medicament for treating: (a) prostate carcinoma, (b) benign prostatic hyperplasia, (c) hirsutism, (d) alopecia, (e) anorexia nervosa, (f) breast cancer, (g) acne, (h) AIDS, (i) cachexia, for (j) male contraception, or for (k) male performance enhancement, in a subject in need thereof.

### DETAILED DESCRIPTION OF THE INVENTION

The present invention is directed to compounds of formula (I) 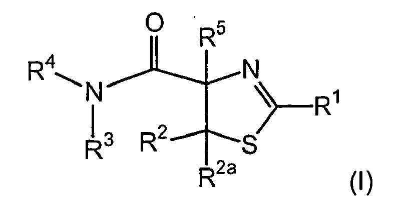 wherein R¹, R², R^{2a}, R³, R⁴ and R are as herein defined, useful as selective androgen receptor modulators for the treatment of prostate carcinoma, benign prostatic hyperplasia (BPH), hirsitutism, alopecia, anorexia nervosa, breast cancer, acne, AIDS, cachexia, as a male contraceptive, and as a male performance enhancer.

In an embodiment of the present invention R¹ is aryl, wherein the aryl group is optionally substituted with one to two substituents independently selected from halogen, lower alkyl, halogen substituted lower alkyl, lower alkyl ester, cyano, N(R^{A})₂C(O)-, lower alkyl-C(O)-NR^{A}-, lower alkyl-S(O)₀₋₂-, phenyl-S(O)₀₋₂, lower alkyl-S(O)₀₋₂-NR^{A}-, phenyl-S(O)₀₋₂-NR^{A}- and trifluoromethyl-sulfonyl-; and wherein the phenyl is optionally substituted with one or more substitutents independently selected from halogen, lower alkyl, lower alkoxy, hydroxy, carboxy, cyano, nitro, amino, lower alkylamino or di(lower alkyl)amino.

In another embodiment of the present invention, R¹ is heteroaryl, wherein the heteroaryl group is optionally substituted with one to two substituents independently selected from halogen, lower alkyl, halogen substituted lower alkyl, lower alkyl ester, cyano, N(R^{A})₂C(O)-, lower alkyl-C(O)-NR^{A}-, lower alkyl-S(O)₀₋₂-, phenyl-S(O)₀₋₂, lower alkyl-S(O)₀₋₂-NR^{A}-, phenyl-S(O)₀₋₂-NR^{A}- and trifluoromethyl-sulfonyl-; and wherein the phenyl is optionally substituted with one or more substitutents independently selected from halogen, lower alkyl, lower alkoxy, hydroxy, carboxy, cyano, nitro, amino, lower alkylamino or di(lower alkyl)amino.

In another embodiment of the present invention, R¹ is aryl, wherein the aryl group is optionally substituted with a halogen. Preferably, R¹ is selected from the group consisting of 4-fluorophenyl, 3-fluorophenyl, 4-chlorophenyl and 3-chlorophenyl. More preferably, R¹ is selected from the group consisting of 4-fluorophenyl, 3-chlorophenyl and 4-chlorophenyl. More preferably still, R¹ is selected from the group consisting of 4-fluorophenyl and 4-chlorophenyl.

In an embodiment of the present invention R^{A} is hydrogen or methyl.

In an embodiment of the present invention, R² is selected from the group consisting of hydrogen, lower alkyl and trifluoromethyl. Preferably R² is selected from the group consisting of hydrogen, methyl and trifluoromethyl. More preferably, R² is hydrogen or methyl. More preferably still, R² is hydrogen.

In an embodiment of the present invention, R^{2a} is selected from the group consisting of hydrogen, lower alkyl and trifluoromethyl. Preferably R^{2a} is selected from the group consisting of hydrogen, methyl and trifluoromethyl. More preferably, R^{2a} is hydrogen or methyl. More preferably still, R^{2a} is hydrogen.

In an embodiment of the present invention, R³ is selected from the group consisting of hydrogen and lower alkyl, preferably R³ is selected from the group consisting of hydrogen and methyl; more preferably R³ is hydrogen.

In an embodiment of the present invention R⁴ is aryl, wherein the aryl group is optionally substituted with one to three substituents independently selected from halogen, lower alkyl, halogen substituted lower alkyl, lower alkyl ester, cyano, N(R^{A})₂C(O)-, lower alkyl-C(O)-NR^{A}-, lower alkyl-S(O)₀₋₂-, phenyl-S(O)₀₋₂, lower alkyl-S(O)₀₋₂-NR^{A}-, phenyl-S(O)₀₋₂-NR^{A}- and trifluoromethyl-sulfonyl-; and wherein the phenyl is optionally substituted with one or more substitutents independently selected from halogen, lower alkyl, lower alkoxy, hydroxy, carboxy, cyano, nitro, amino, lower alkylamino or di(lower alkyl)amino.

In another embodiment of the present invention, R⁴ is heteroaryl, wherein the heteroaryl group is optionally substituted with one to two substituents independently selected from halogen, lower alkyl, halogen substituted lower alkyl, lower alkyl ester, cyano, N(R^{A})₂C(O)-, lower alkyl-C(O)-NR^{A}-, lower alkyl-S(O)₀₋₂-, phenyl-S(O)₀₋₂, lower alkyl-S(O)₀₋₂-NR^{A}-, phenyl-S(O)₀₋₂-NR^{A}- and trifluoromethyl-sulfonyl-; and wherein the phenyl is optionally substituted with one or more substitutents independently selected from halogen, lower alkyl, lower alkoxy, hydroxy, carboxy, cyano, nitro, amino, lower alkylamino or di(lower alkyl)amino.

In another embodiment of the present invention, R⁴ is aryl, wherein the aryl group is substituted with one to three substituents independently selected from halogen, cyano, nitro, lower alkyl, halogen substituted lower alkyl and trifluoromethyl-sulfonyl. Preferably, R⁴ is selected from the group consisting of 4-chlorophenyl, 4-cyanophenyl, 3-trifluoromethyl-4-cyano-phenyl, 3-trifluoromethyl-4-nitro-phenyl, 3-trifluoromethyl-4-chloro-phenyl, 3-trifluoromethyl-4-fluoro-phenyl, 2,5-difuoro-4-cyano-phenyl, 2-fluoro-3-trifluoromethyl-phenyl and 4-trifluoromethyl-sulfonyl-phenyl. More preferably, R⁴ is selected from the group consisting of 3-trifluoromethyl-4-cyano-phenyl, 3-trifluoromethyl-4-nitro-phenyl, 3-trifluoromethyl-4-chloro-phenyl and 4-trifluoromethylsulfonyl-phenyl. More preferably still, R⁴ is selected from the group consisting of 3-trifluoromethyl-4-nitro-phenyl and 3-trifluoromethyl-4-chloro-phenyl.

In an embodiment of the present invention, R⁵ is selected from the group consisting of hydrogen, lower alkyl and trifluoromethyl. Preferably R⁵ is selected from the group consisting of hydrogen, methyl and trifluoromethyl. More preferably, R⁵ is hydrogen or methyl. More preferably still, R⁵ is selected from the group consisting of hydrogen and methyl.

Representative compounds of the instant invention were prepared from L-amino acids according to the processes described herein and are as listed in Table 1 below. Stereoconfiguration of the final product was not determined.

**Table 1**

| | | | | | |
|---|---|---|---|---|---|
| 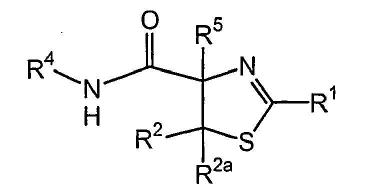 | | | | | |

| **Cmpd #** | **R¹** | **R²** | **R^{2a}** | **R⁵** | **R⁴** |
|---|---|---|---|---|---|
| 1 | 4-fluoro-phenyl | H | H | H | 3-trifluoro-methyl-4-cyano-phenyl |
| 2 | 4-fluoro-phenyl | H | H | H | 4-cyano-phenyl |
| 3 | 3-fluoro-phenyl | H | H | H | 3-trifluoro-methyl-4-cyano-phenyl |
| 4 | 4-fluoro-phenyl | H | H | H | 3-trifluoro-methyl-4-nitro-phenyl |
| 5 | 4-fluoro-phenyl | H | H | H | 3-trifluoro-methyl-4-chloro-phenyl |
| 7 | 4-fluoro-phenyl | H | H | H | 3-trifluoro-methyl-4-fluoro-phenyl |
| 9 | 4-chloro-phenyl | H | H | H | 3-trifluoro-methyl-4-cyano-phenyl |
| 10 | 4-chloro-phenyl | H | H | H | 3-trifluoro-methyl-4-nitro-phenyl |
| 11 | 4-chloro-phenyl | H | H | H | 3-trifluoro-methyl-4-chloro-phenyl |
| 12 | 4-fluoro-phenyl | H | H | methyl | 3-trifluoro-methyl-4-chloro-phenyl |
| 13 | 4-fluoro-phenyl | H | H | methyl | 3-trifluoro-methyl-4-nitro-phenyl |
| 14 | 4-chloro-phenyl | H | H | H | 3-trifluoro-methyl-4-fluoro-phenyl |
| 15 | 4-chloro-phenyl | H | H | H | 3-trifluoro-methyl-4-fluoro-phenyl |
| 16 | 4-chloro-phenyl | H | H | H | 4-chloro-phenyl |
| 17 | 3-fluoro-phenyl | H | H | H | 3-trifluoro-methyl-4-chloro-phenyl |
| 18 | 3-fluoro-phenyl | H | H | H | 3-trifluoro-methyl-4-fluoro-phenyl |
| 19 | 4-fluoro-phenyl | methyl | methyl | H | 3-trifluoro-methyl-4-cyano-phenyl |
| 20 | 4-fluoro-phenyl | methyl | methyl | H | 3-trifluoro-methyl-4-nitro-phenyl |
| 21 | 4-fluoro-phenyl | methyl | methyl | H | 3-trifluoro-methyl-4-chloro-phenyl |
| 22 | 4-chloro-phenyl | methyl | methyl | H | 3-trifluoro-methyl-4-chloro-phenyl |
| 23 | 4-chloro-phenyl | methyl | methyl | H | 2,5-difluoro-4-cyano-phenyl |
| 24 | 4-chloro-phenyl | H | H | H | 2-fluoro-3-trifluoro-methyl-phenyl |
| 25 | 4-fluoro-phenyl | H | H | H | 2-fluoro-3-trifluoro-methyl-phenyl |
| 26 | 3-chloro-phenyl | H | H | H | 3-trifluoro-methyl-4-chloro-phenyl |
| 27 | 3-chloro-phenyl | H | H | H | 3-trifluoro-methyl-4-cyano-phenyl |
| 28 | 4-fluoro-phenyl | H | H | H | 4-trifluoro-methyl-sulfonyl-phenyl |
| 29 | 4-chloro-phenyl | H | H | H | 4-trifluoro-methyl-sulfonyl-phenyl |
| 30 | 3-chloro-phenyl | H | H | H | 3-trifluoro-methyl-4-nitro-phenyl |
| 31 | 3-chloro-phenyl | H | H | H | 4-trifluoro-methyl-sulfonyl-phenyl |
| 32 | 3-chloro-phenyl | H | H | H | 2-fluoro-3-trifluoro-methyl-phenyl |
| 33 | 4-fluoro-phenyl | H | H | methyl | 3-trifluoro-methyl-4-cyano-phenyl |

As used herein, "halogen" shall mean chlorine, bromine, fluorine and iodine.

As used herein, the term "alkyl" whether used alone or as part of a substituent group, include straight and branched chains. For example, alkyl radicals include methyl, ethyl, propyl, isopropyl, butyl, isobutyl, sec-butyl, t-butyl, pentyl and the like. Unless otherwise noted, "lower" when used with alkyl means a carbon chain composition of 1-4 carbon atoms.

As used herein, unless otherwise noted, the term "halogen substituted lower alkyl" shall mean a lower alkyl group as defined above wherein one or more of the hydrogen atoms is replaced with halogen atoms. Suitable examples include, but are not limited to trifluoromethyl, 1,1,1-trifluoro-ethyl, chloromethyl, fluoromethyl and the like.

As used herein, unless otherwise noted, "alkoxy" shall denote an oxygen ether radical of the above described straight or branched chain alkyl groups. For example, methoxy, ethoxy, n-propoxy, sec-butoxy, t-butoxy, n-hexyloxy and the like.

As used herein, unless otherwise noted, "aryl" shall refer to unsubstituted carbocylic aromatic groups such as phenyl, naphthyl, and the like.

As used herein, unless otherwise noted, "heteroaryl" shall denote any five or six membered monocyclic aromatic ring structure containing at least one heteroatom selected from the group consisting of O, N and S, optionally containing one to three additional heteroatoms independently selected from the group consisting of O, N and S; or a nine or ten membered bicyclic aromatic ring structure containing at least one heteroatom selected from the group consisting of O, N and S, optionally containing one to four additional heteroatoms independently selected from the group consisting of O, N and S. Examples of suitable heteroaryl groups include, but are not limited to, pyrrolyl, furyl, thienyl, oxazolyl, imidazolyl, purazolyl, isoxazolyl, isothiazolyl, triazolyl, thiadiazolyl, pyridyl, pyridazinyl, pyrimidinyl, pyrazinyl, pyranyl, furazanyl, indolizinyl, indolyl, isoindolinyl, indazolyl, benzofuryl, benzothienyl, benzimidazolyl, benzthiazolyl, purinyl, quinolizinyl, quinolinyl, isoquinolinyl, isothiazolyl, cinnolinyl, phthalazinyl, quinazolinyl, quinoxalinyl, naphthyridinyl, pteridinyl, and the like.

As used herein, the notation "*" shall denote the presence of a stereogenic center.

When a particular group is "substituted" (e.g., Phe, aryl, heteroalkyl, heteroaryl), that group may have one or more substituents, preferably from one to five substituents, more preferably from one to three substituents, most preferably from one to two substituents, independently selected from the list of substituents.

With reference to substituents, the term "independently" means that when more than one of such substituents is possible, such substituents may be the same or different from each other.

Under standard nomenclature used throughout this disclosure, the terminal portion of the designated side chain is described first, followed by the adjacent functionality toward the point of attachment. Thus, for example, a "phenylC₁-C₆alkylaminocarbonylC₁-C₆alkyl" substituent refers to a group of the formula

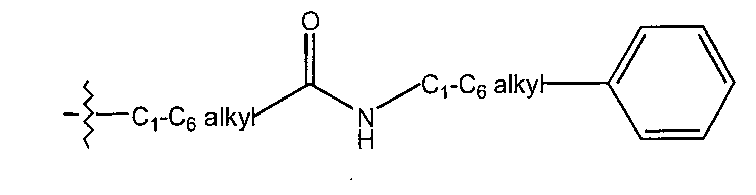

Under the standard nomenclature used throughout this disclosure substituents on the core thiazoline group will be designated as bound at the following positions of attachment:

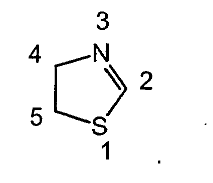

Abbreviations used in the specification, particularly the Schemes and Examples, are as follows:

| | | |
|---|---|---|
| DCE | = | Dichloroethane |
| DIPEA or DIEA or *i*Pr₂NEt | = | Diisopropylethylamine |
| DMAP | = | 4-N,N-Dimethylaminopyridine |
| DMF | = | N,N-Dimethylformamide |
| DMPU | = | 1,3-Dimethyl-3,4,5,6-tetrahydro-2(1H)-pyrimidine |
| Et | = | Ethyl |
| Et₃N | = | Triethylamine |
| EtOAc | = | Ethyl Acetate |
| *i*Pr | = | Isopropyl |
| PyBrOP | = | Bromo-tris-pyrrolidino-phosphonium hexafluorophosphate |
| TEA | = | Triethylamine |
| THF | = | Tetrahydrofuran |

The term "subject" as used herein, refers to an animal, preferably a mammal, most preferably a human, who has been the object of treatment, observation or experiment.

The term "therapeutically effective amount" as used herein, means that amount of active compound or pharmaceutical agent that elicits the biological or medicinal response in a tissue system, animal or human that is being sought by a researcher, veterinarian, medical doctor or other clinician, which includes alleviation of the symptoms of the disease or disorder being treated.

As used herein, the term "composition" is intended to encompass a product comprising the specified ingredients in the specified amounts, as well as any product which results, directly or indirectly, from combinations of the specified ingredients in the specified amounts.

Where the compounds according to this invention have at least one chiral center, they may accordingly exist as enantiomers. Where the compounds possess two or more chiral centers, they may additionally exist as diastereomers. It is to be understood that all such isomers and mixtures thereof are encompassed within the scope of the present invention. Furthermore, some of the crystalline forms for the compounds may exist as polymorphs and as such are intended to be included in the present invention. In addition, some of the compounds may form solvates with water (i.e., hydrates) or common organic solvents, and such solvates are also intended to be encompassed within the scope of this invention.

Where the processes for the preparation of the compounds according to the invention give rise to mixture of stereoisomers, these isomers may be separated by conventional techniques such as preparative chromatography. The compounds may be prepared in racemic form, or individual enantiomers may be prepared either by enantiospecific synthesis or by resolution. The compounds may, for example, be resolved into their component enantiomers by standard techniques, such as the formation of diastereomeric pairs by salt formation with an optically active acid, such as (-)-di-p-toluoyl-D-tartaric acid and/or (+)-di-p-toluoyl-L-tartaric acid followed by fractional crystallization and regeneration of the free base. The compounds may also be resolved by formation of diastereomeric esters or amides, followed by chromatographic separation and removal of the chiral auxiliary. Alternatively, the compounds may be resolved using a chiral HPLC column.

During any of the processes for preparation of the compounds of the present invention, it may be necessary and/or desirable to protect sensitive or reactive groups on any of the molecules concerned. This may be achieved by means of conventional protecting groups, such as those described in Protective Groups in Organic Chemistry, ed. J.F.W. McOmie, Plenum Press, 1973; and T.W. Greene & P.G.M. Wuts, Protective Groups in Organic Synthesis, John Wiley & Sons, 1991. The protecting groups may be removed at a convenient subsequent stage using methods known from the art.

Compounds of formula (I) wherein R⁵ is hydrogen may be prepared according to the process outlined in Scheme 1.

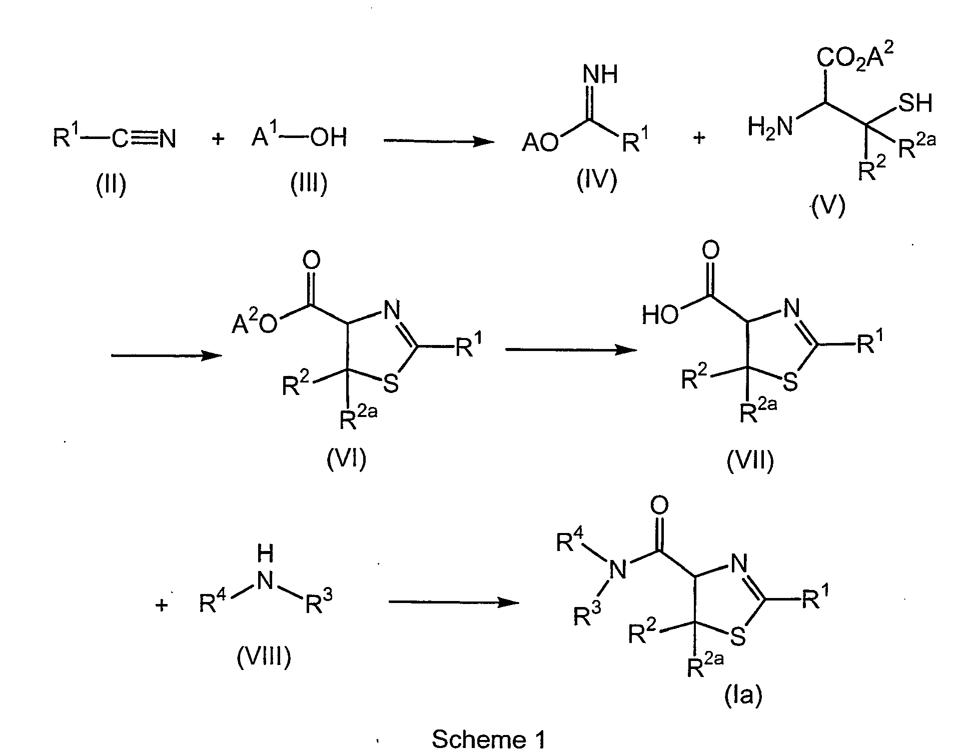

Accordingly, a suitably substituted compound of formula (II), a known compound or compound prepared by known methods, is reacted with a compound of formula (III), wherein A¹ is a lower alkyl, a known compound or compound prepared by known methods, in the presence of an acid such as hydrochloric acid, sulfuric acid, and the like, optionally in an organic solvent such as diethyl ether, and the like, to promote solubility, to yield the corresponding compound of formula (IV)

The compound of formula (IV) is reacted with a suitably substituted compound of formula (V), wherein A² is a lower alkyl, a known compound or compound prepared by known methods, in the presence of a base such as TEA, DIPEA, pyridine, and the like, in an organic solvent such as DCE, methylene chloride, THF, and the like, to yield the corresponding compound of formula (VI).

The compound of formula (VI) is reacted with a base such as KOH, NaOH, LiOH, and the like, in an organic solvent such as THF, dioxane, water, and the like, in the presence of water, to yield the corresponding compound of formula (VII).

The compound of formula (VII) is reacted with a suitably substituted compound of formula (VIII), a known compound or compound prepared by known methods, in the presence of a catalyst or catalyst pair such as PyBrOP and DMAP, DCC and DMAP, EDCI and HOBT, and the like, in the presence of a base such as DIPEA, TEA, pyridine, and the like, in an aprotic solvent such as DMF, DCE, DCM, and the like, preferably in an anhydrous aprotic solvent, to yield the corresponding compound of formula (Ia).

Compounds of formula (I) wherein R⁵ is other than hydrogen may be prepared according to the process outlined in Scheme 2.

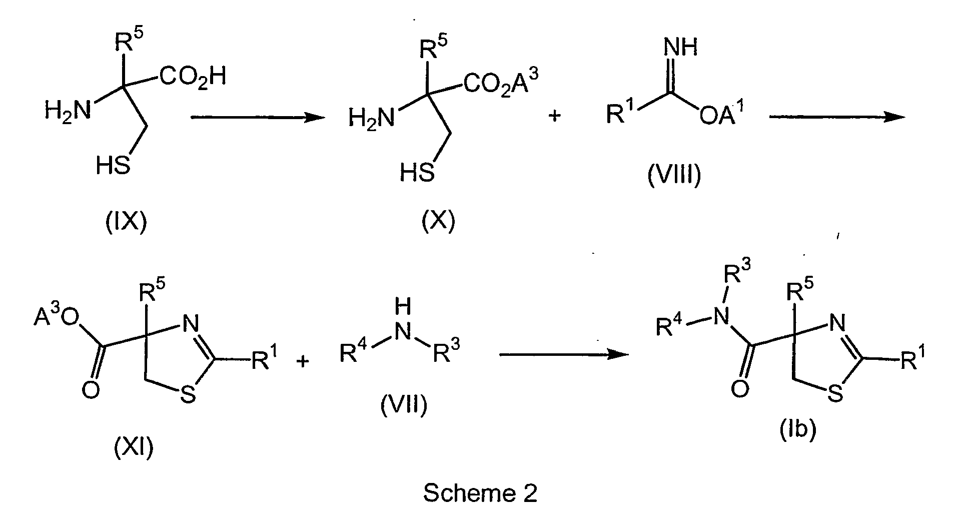

Accordingly, a compound of formula (IX), wherein a known compound or compound prepared by known methods (for example, according to the process disclosed by Pattende, G., et al., Tetrahedron, (1993), 49(10), pp2131-2138) is reacted with thionyl chloride, wherein A³ is a lower alkyl, in an alcoholic solvent, to yield the corresponding compound of formula (X).

The compound of formula (X) is reacted with a suitably substituted compound of formula (VIII), wherein A¹ is a lower alkyl, a known compound or compound prepared by known methods, in the presence of a base such as TEA, DIPEA, pyridine, and the like, in an organic solvent such as DCE, methylene chloride, THF, and the like, to yield the corresponding compound of formula (XI).

The compound of formula (XI) is reacted with a suitably substituted compound of formula (VIII), a known compound or compound prepared by known methods, in the presence of a catalyst or catalyst pair such as PyBrOP and DMAP, DCC and DMAP, EDCI and HOBT, and the like, in the presence of a base such as DIPEA, TEA, pyridine, and the like, in an aprotic solvent such as DMF, DCE, DCM, and the like, preferably in an anhydrous aprotic solvent, to yield the corresponding compound of formula (Ia).

One skilled in the art will recognize that compound of formula (I) wherein R² and/or R^{2a} are other than hydrogen, may b similarly prepared according to the process outlined in Scheme 2 above, with substitution of a suitably substituted compound of formula (XII) 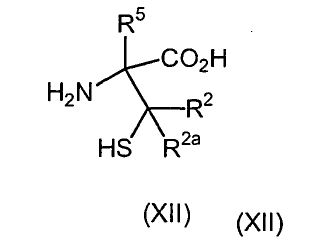 a known compound or compound prepared by known methods, for the compound of formula (IX).

The following Examples are set forth to aid in the understanding of the invention, and are not intended and should not be construed to limit in any way the invention set forth in the claims which follow thereafter.

### Example 1 (Cmpd #9)

### 2-(4-chloro-phenyl)-4,5-dihydro-thiazole-4-carboxylic acid (4-cyano-3-trifluoromethyl-phenyl)-amide

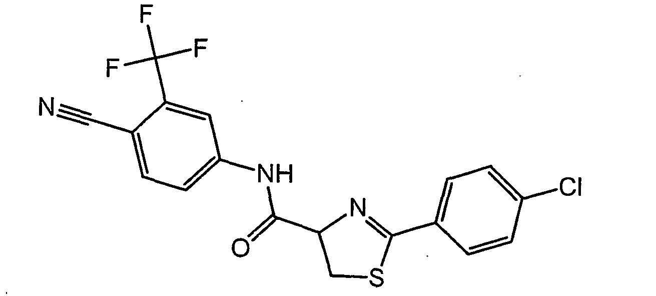

### Step A: 4-chloro-benzimidic acid ethyl ester hydrochloride

To a solution of 4-chlorobenzonitrile (11.36g, 85.6 mmol) in ethanol (60 mL) was bubbled in dry HCl (200 mL 36% HCl was added dropwise into 500 mL of conc. H₂SO₄) at 5°C. The reaction mixture was stirred for an additional 0.5 hour at 0°C and then diluted with anhydrous diethyl ether (500 mL). After the mixture was stirred at 5°C for 2 hours, filtered, washed with diethyl ether, dried under vacuum to yield 4-chloro-benzimidic acid ethyl ester hydrochloride. The product was carried on to the next step without further purification.

### Step B: 2-(4-chloro-phenyl)-4,5-dihydro-thiazole-4-carboxylic acid methyl ester

Triethylamine (2.44g, 24.16 mmol) was added dropwise into a solution of 4-chloro-benzimidic acid ethyl ester hydrochloride (5 g, 22.71 mmol) and L-cysteine methyl ester hydrochloride (4.29g, 24.98 mmol) in dichloromethane (85 mL) at 0°C. The resulting mixture was stirred at room temperature for 3 days. Water and dichloromethane were added, and the water layer was extracted with dichloromethane, dried over Na₂SO₄, and concentrated in vacuum to yield the crude product. Purification by column chromatography (silica gel, 15% EtOAc/hexane) yielded 2-(4-chloro-phenyl)-4,5-dihydro-thiazole-4-carboxylic acid methyl ester.

### Step C: 2-(4-chloro-phenyl)-4,5-dihydro-thiazole-4-carboxylic acid

To a solution of 2-(4-chlorophenyl)-4,5-dihydro-thiazole-4-carboxylic acid methyl ester (5.23g, 0.02 mole) in THF (200 mL) and H₂O (200 mL) was added KOH (11.4g, 0.2 mole). The reaction mixture was vigorously stirred at room temperature for 17 hours, then diluted with water, extracted with diethyl ether and the diethyl ether layer washed with water. The combined water layers were acidified with 1N HCl to pH 1, extracted with diethyl ether and dried over anhydrous Na₂SO₄. The dessicant was filtered off and the filtrate was concentrated under vacuum to yield 2-(4-chlor-phenyl)-4,5-dihydro-thiazole-4-carboxylic acid.
¹H NMR (400 MHz, d₆-DMSO): δ 13.03 (br s, 1H), 7.81 (d, 2H, *J* = 8.5 Hz), 7.57 (d, 2H, *J* = 8.5 Hz), 5.31 (dd, 1H, *J* = 8.3, 9.4 Hz), 3.62 - 3.77 (m, 2H).

### Step D: 2-(4-chloro-phenyl)-4,5-dihydro-thiazole-4-carboxylic acid (4-cyano-3-trifluoromethyl-phenyl)-amide

To a solution of 2-(4-chlorphenyl)-4,5-dihydro-thiazole-4-carboxylic acid (186 mg, 0.770 mmol) and 5-amino-2-cyanobenzotrifluoride (158 mg, 0.847 mmol) in DMF (10 mL) were added DMAP (94 mg, .770 mmol)), *i*Pr₂NEt (0.25 mL), and PyBroP (1.08g, 2.311 mmol) sequentially. The reaction mixture was stirred at room temperature overnight. Water and EtOAc were added. The EtOAc layer was washed with 1N HCl and brine, then dried over anhydrous Na₂SO₄. The dessicant was filtered off and the filtrate was concentrated under vacuum to yield crude product. The crude product was purified by column chromatography (silica gel, 20% EtOAc/hexane) to yield 2-(4-chloro-phenyl)-4,5-dihydro-thiazole-4-carboxylic acid (4-cyano-3-trifluoromethyl-phenyl)-amide.
¹H NMR (400 MHz, CDCl₃): δ 9.06 (br s, 1H), 8.04 (d,1H, *J* = 1.9 Hz), 7.98 (dd, 1H, *J* = 2.0, 8.5 Hz), 7.84 (d, 2H, *J* = 8.5 Hz), 7.45 - 7.85 (m, 1H), 7.46 (d, 2H, *J* = 8.5 Hz), 5.34 (t, 1H, *J* = 10 Hz), 3.73 - 3.88 (m, 2H)
MS m/z (M-H) = 408.

### Example 2 (Cpmd #3)

### 2-(3-fluoro-phenyl)-4,5-dihydro-thiazole-4-carboxylic acid (4-cyano-3-trifluoromethyl-phenyl)-amide

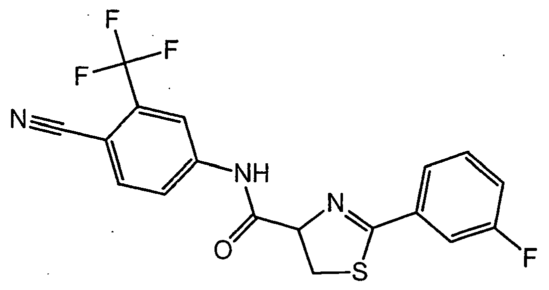

### Step A: 3-fluoro-benzimidic acid ethyl ester hydrochloride

To a solution of 3-fluorobenzonitrile (10 g, 82.6 mmol) in ethanol (20 mL) was bubbled in dry HCl (200 mL of 36% HCl was added dropwise into 500 mL of conc. H₂SO₄) at 5°C. The reaction mixture was stirred for an additional 0.5 hour at 0°C and then diluted with anhydrous ether (500 mL). The reaction mixture was then stirred at 5°C for 2 hours, filtered, washed with diethyl ether and dried under vacuum to yield 3-fluoro-benzimidic acid ethyl ester hydrochloride. The product was carried on to the next step without further purification.

### Step B: 2-(3-fluoro-phenyl)-4,5-dihydro-thiazole-4-carboxylic acid methyl ester

Triethylamine (1.1 ml) was added dropwise into a solution of 3-fluoro-benzimidic acid ethyl ester hydrochloride (1.5 g, 7.366 mmol) and L-cysteine methyl ester hydrochloride (1.39g, 8.102 mmol) in dichloromethane (25 mL) at 0°C. The resulting mixture was stirred at room temperature for 2 days. Water and dichloromethane were added, and the water layer was extracted with dichloromethane, dried over Na₂SO₄, and concentrated in vacuum to yield crude product. The crude product was purified by column chromatography (silica gel, 15% EtOAc/hexane) to yield 2-(3-fluoro-phenyl)-4,5-dihydro-thiazole-4-carboxylic acid methyl ester.

### Step C: 2-(3-fluoro-phenyl)-4,5-dihydro-thiazole-4-carboxylic acid (4-cyano-3-trifluoromethyl-phenyl)-amide

To a solution of 2-(3-fluoro-phenyl)-4,5-dihydro-thiazole-4-carboxylic acid methyl ester (274 mg, 1.145 mmol) and 5-amino-2-cyano-benzonitrile (426 mg, 2.287 mmol) in THF (5 mL) at 0°C was added 2M isopropyl magnesium chloride (1.14 mL in THF). The reaction mixture was stirred at room temperature overnight. The reaction mixture was diluted with water and EtOAc. The organic layer was washed with 1N HCl and brine, then dried over anhydrous Na₂SO₄. The dessicant was filtered off and the filtrate was concentrated under vacuum to yield crude product. The crude product was purified by column chromatography (silica gel, 20% EtOAc/hexane) to yield 2-(3-fluoro-phenyl)-4,5-dihydro-thiazole-4-carboxylic acid (4-cyano-3-trifluoromethyl phenyl)-amide.
¹H NMR (400 MHz, CDCl₃): δ 9.05 (br s, 1H), 8.07 (d, 1H, *J* = 2.0 Hz), 7.98 (dd, 1H, *J* = 2.0, 8.5 Hz), 7.81 (d, 1H, *J* = 8.5 Hz), 7.62 - 7.67 (m, 2H), 7.43 - 7.50 (m, 1H), 7.23 - 7.30 (m, 1H), 5.35 (t, 1H, *J* = 10 Hz), 3.73 - 3.90 (m, 2H)
MS m/z (M-H) = 391

### Example 3 (Cmpd #17)

### 2-(3-fluoro-phenyl)-4,5-dihydro-thiazole-4-carboxylic acid (4-chloro-3-trifluoromethyl-phenyl)-amide

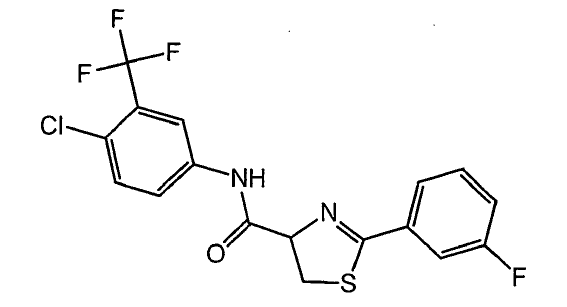

To a solution of 2-(3-fluoro-phenyl)-4,5-dihydro-thiazole-4-carboxylic acid (178 mg, 0.79 mmol) and 5-amino-2-chloro-benzonitrile (155 mg, 0.79 mmol) in DMF (10 mL) were added DMAP (97 mg, 0.79 mmol), *i*Pr₂NEt (0.28 mL), and PyBroP (553 mg, 1.185 mmol) sequentially. The reaction mixture was stirred at room temperature overnight. The reaction mixture was diluted with water and extracted with EtOAc. The organic extracts were washed with 1N HCl and brine, then dried over anhydrous Na₂SO₄. The dessicant was filtered off and the filtrate was concentrated under vacuum to yield crude product. The crude product was purified by column chromatography (silica gel, 20% EtOAc/hexane) to yield 2-(3-fluoro-phenyl)-4,5-dihydro-thiazole-4-carboxylic acid (4-chloro-3-trifluoromethyl-phenyl)-amide.
¹H NMR (300 MHz, CDCl₃): d 8.83 (br s, 1H), 7.93 (d, 1H, *J* = 2.6 Hz), 7.80 (dd,1H, *J* = 2.6, 8.9 Hz), 7.62-7.67 (m, 2H), 7.41-7.49 (m, 3H), 7.25-7.28 (m, 1H), 5.33 (t, 1H, *J* = 9.9 Hz), 3.73 - 3.89 (m, 2H).

### Example 4 (Cmpd #18)

### 2-(3-fluoro-phenyl)-4,5-dihydro-thiazole-4-carboxylic acid (4-fluoro-3-trifluoromethyl-phenyl)-amide

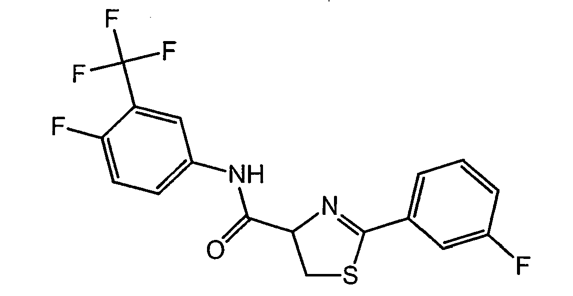

To a solution of 2-(3-fluoro-phenyl)-4,5-dihydro-thiazole-4-carboxylic acid (187 mg, 0.83 mmol) and 5-amino-2-fluoro-benzonitrile (149 mg, 0.83 mmol) in DMF (10 mL) were added DMAP (104 mg, 0.83 mmol), *i*Pr₂NEt (0.29 mL), and PyBroP (581 mg, 1.24 mmol) sequentially. The reaction mixture was stirred at room temperature overnight. The reaction mixture was diluted with water and extracted with EtOAc. The organic extracts were washed with 1N HCl and brine, then dried over anhydrous Na₂SO₄. The dessicant was filtered off and the filtrate was concentrated under vacuum to yield crude product. The crude product was purified by column chromatography (silica gel, 20% EtOAc/hexane) to yield 2-(3-fluoro-phenyl)-4,5-dihydro-thiazole-4-carboxylic acid (4-fluoro-3-trifluoromethyl-phenyl)-amide.

¹H NMR (300 MHz, CDCl₃): d 8.77 (br s, 1H), 7.15 - 8.01 (m, 7H), 5.33 (t, 1H, *J* = 10 Hz), 3.73 - 3.89 (m, 2H).

### Example 5 (Cmpd #9)

### 2-(4-chloro-phenyl)-4,5-dihydro-thiazole-4-carboxylic acid (4-cyano-3-trifluoromethyl-phenyl)-amide

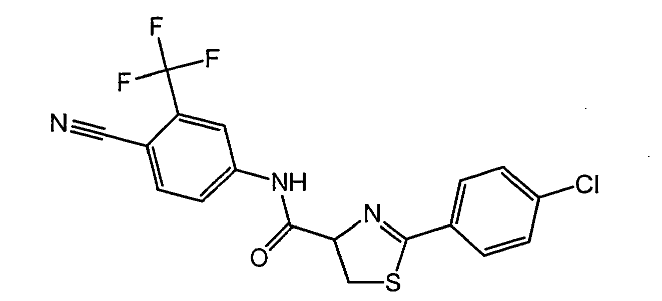

### Step A: 2-(4-chloro-phenyl)-4,5-dihydro-thiazole-4-carboxylic acid

To a solution of 2-(4-chlorophenyl)-4,5-dihydro-thiazole-4-carboxylic acid methyl ester (5.23g, 0.02 mole) in THF (200 mL) and H₂O (200 mL) was added KOH (11.4g, 0.2 mole). The reaction mixture was vigorously stirred at room temperature for 17 hours. The reaction mixture was then diluted with water, extracted with diethyl ether and the diethyl ether layer was washed with water. The combined water layers were acidified with 1N HCl to pH 1, then extracted with diethyl ether and dried over anhydrous Na₂SO₄. The dessicant was filtered off and the filtrate was concentrated under vacuum to yield 2-(4-chlorphenyl)-4,5-dihydro-thiazole-4-carboxylic acid.
¹H NMR (400 MHz, d₆-DMSO): δ 13.03 (br s, 1H), 7.81 (d, 2H, *J* = 8.5 Hz), 7.57 (d, 2H, *J* = 8.5 Hz), 5.31 (dd, 1H, *J* = 8.3, 9.4 Hz), 3.62 - 3.77 (m, 2H).

### Step B: 2-(4-chloro-phenyl)-4,5-dihydro-thiazole-4-carboxylic acid (4-cyano-3-trifluoromethyl-phenyl)-amide

To a solution of 2-(4-chloro-phenyl)-4,5-dihydro-thiazole-4-carboxylic acid (186 mg, 0.770 mmol) and 5-amino-2-cyanobenzotrifluoride (158 mg, 0.847 mmol) in DMF (10 mL) were added DMAP (94 mg, .770 mmol)), *i*Pr₂NEt (0.25 mL), and PyBroP (1.08g 2.311 mmol) sequentially. The reaction mixture was stirred at room temperature overnight. Water and EtOAc was added. EtOAc layer was washed with 1N HCl and brine, then dried over anhydrous Na₂SO₄. The dessicant was filtered off and the filtrate was concentrated under vacuum to yield crude product. The crude product was purified by column chromatography (silica gel, 20% EtOAc/hexane) to yield 2-(4-chloro-phenyl)-4,5-dihydro-thiazole-4-carboxylic acid (4-cyano-3-trifluoromethyl-phenyl)-amide.
¹H NMR (400 MHz, CDCl₃): δ 9.06 (br s, 1H), 8.04 (d, 1H, *J* = 1.9 Hz), 7.98 (dd, 1H, *J* = 2.0, 8.5 Hz), 7.84 (d, 2H, *J* = 8.5 Hz), 7.45 - 7.85 (m, 1H), 7.46 (d, 2H; *J* = 8.5 Hz), 5.34 (t, 1H, *J* = 10 Hz), 3.73 - 3.88 (m, 2H)
MS m/z (M-H) = 408.

### Example 6 (Cmpd #11)

### 2-(4-chloro-phenyl)-4,5-dihydro-thiazole-4-carboxylic acid (4-chloro-3-trifluoromethyl-phenyl)-amide

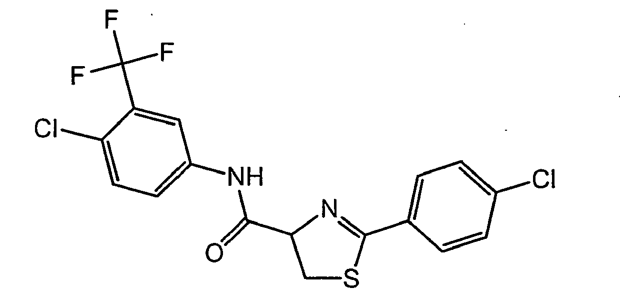

To a solution of 2-(4-chloro-phenyl)-4,5-dihydro-thiazole-4-carbokylic acid (214 mg, 0.8854 mmol) and 5-amino-2-chlorobenzotrifluoride (191 mg, 0.9740 mmol) in DMF (10 mL) were added DMAP (108 mg, .8854 mmol), *i*Pr₂NEt (0.25 mL), and PyBroP (1.24g, 2.6562 mmol) sequentially. The reaction mixture was stirred at room temperature overnight. The reaction mixture was diluted with water and extracted with EtOAc. The organic extracts were washed with 1N HCl and brine, then dried over anhydrous Na₂SO₄. The dessicant was filtered off and the filtrate was concentrated under vacuum to yield crude product. The crude product was purified by column chromatography (silica gel, 20% EtOAc/hexane) to yield 2-(4-chloro-phenyl)-4,5-dihydro-thiazole-4-carboxylic acid (4-chloro-3-trifluoromethyl-phenyl)-amide.
¹H NMR (300 MHz) d 8.79 (br s, 1H), 7.89 - 7.96 (m, 3H), 7.42 - 7.49 (m, 4H), 5.31 (t, 1H, *J* = 10 Hz), 3.71 - 3.89 (m, 2H)
MS m/z (M-H) = 418.

### Example 7 (Cmpd #10)

### 2-(4-chloro-phenyl)-4,5-dihydro-thiazole-4-carboxylic acid (4-nitro-3-trifluoromethyl-phenyl)-amide

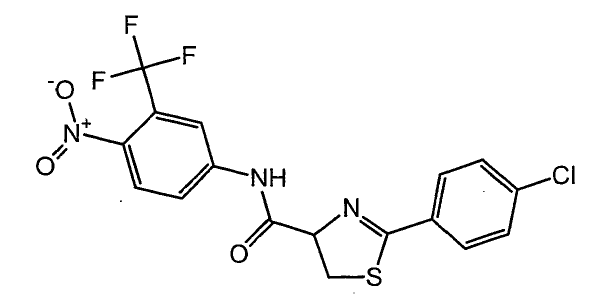

To a solution of 2-(4-chloro-phenyl)-4,5-dihydro-thiazole-4-carboxylic acid (188 mg, 0.7795 mmol) and 5-amino-2-nitrobenzotrifluoride (206 mg, 0.8575 mmol) in DMF (10 mL) were added DMAP (95 mg, .7795 mmol), *i*Pr₂NEt (0.25 mL), and PyBroP (1.09g, 2.3385 mmol) sequentially. The reaction mixture was stirred at room temperature overnight. The reaction mixture was diluted with water and extracted with EtOAc. The organic extracts were washed with 1N HCl and brine, then dried over anhydrous Na₂SO₄. The dessicant was filtered off and the filtrate was concentrated under vacuum to yield crude product. The crude product was purified by column chromatography (silica gel, 20% EtOAc/hexane) to yield 2-(4-nitro-phenyl)-4,5-dihydro-thiazole-4-carboxylic acid (4-nitro-3-trifluoromethyl-phenyl)-amide.
¹H NMR (400 MHz, CDCl₃) δ 9.09 (br s, 1H), 7.98 - 8.07 (m, 3H), 7.85 (d, 2H, *J* = 8.6 Hz), 7.46 (d, 2H, *J* = 8.6 Hz), 5.34 (t, 1H, *J* = 10 Hz), 3.73 - 3.89 (m, 2H)
MS m/z (M-H) 428.

### Example 8 (Cmpd #14)

### 2-(4-chloro-phenyl)-4,5-dihydro-thiazole-4-carboxylic acid (4-fluoro-3-trifluoromethyl-phenyl)-amide

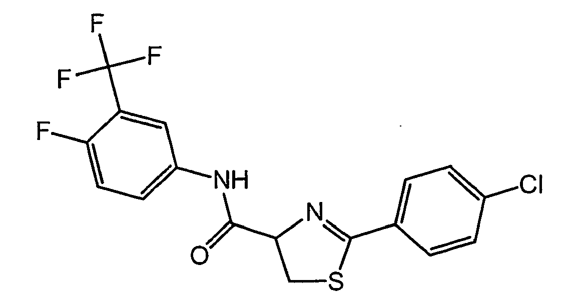

To a solution of 2-(4-chloro-phenyl)-4,5-dihydro-thiazole-4-carboxylic acid (131 mg, 0.5420 mmol) and 5-amino-2-fluorobenzotrifluoride (107 mg, 0.5962 mmol) in DMF (10 mL) were added DMAP (66 mg, .5420 mmol), *i*Pr₂NEt (0.25 mL), and PyBroP (1.76 g, 1.626 mmol) sequentially. The reaction mixture was stirred at room temperature overnight. The reaction mixture was diluted with water and extracted with EtOAc. The organic extracts were washed with 1N HCl and brine, then dried over anhydrous Na₂SO₄. The dessicant was filtered off and the filtrate was concentrated under vacuum to yield crude product. The crude product was purified by column chromatography (silica gel, 20% EtOAc/hexane) to yield 2-(4-chloro-phenyl)-4,5-dihydro-thiazole-4-carboxylic acid (4-fluoro-3-trifluoromethyl-phenyl)-amide.
MS m/z (M-H) 401.

### Example 9 (Cmpd #16)

### 2-(4-chloro-phenyl)-4,5-dihydro-thiazole-4-carboxylic acid (4-chloro-phenyl)-amide

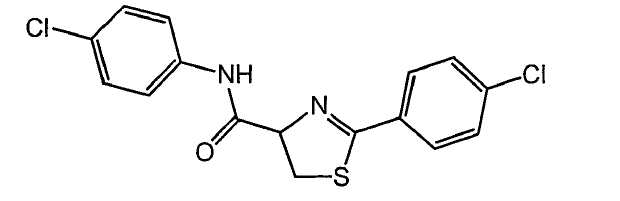

To a solution of 2-(4-chloro-phenyl)-4,5-dihydro-thiazole-4-carboxylic acid (151 mg, 0.6260 mmol) and 4-chloroaniline (76.5 mg, 0.6886 mmol) in DMF (10 mL) were added DMAP (76.5 mg, .626 mmol), *i*Pr₂NEt (0.25 mL), and PyBroP (1.18 g, 2.521 mmol) sequentially. The reaction mixture was stirred at room temperature overnight. The reaction mixture was diluted with water and extracted with EtOAc. The organic extracts were washed with 1N HCl and brine, then dried over anhydrous Na₂SO₄. The dessicant was filtered off and the filtrate was concentrated under vacuum to yield crude product. The crude product was purified by column chromatography (silica gel, 20% EtOAc/hexane) to yield 2-(4-chloro-phenyl)-4,5-dihydro-thiazole-4-carboxylic acid (4-chloro-phenyl)-amide.
¹H NMR (400 MHz, CDCl₃): δ 8.63 (br s, 1H), 7.84 (d, 2H, *J* = 8.5 Hz), 7.53 - 7.57 (m, 2H), 7.44 (d, 2H, *J* = 8.5 Hz), 7.01 - 7.07 (m, 1H), 5.31 (t, 1H, *J* = 9.9 Hz), 3.74-3.87 (m, 2H)
MS m/z (M-H) = 333.

### Example 10 (Cmpd #24)

### 2-(4-chloro-phenyl)-4,5-dihydro-thiazole-4-carboxylic acid (2-fluoro-3-trifluoromethyl-phenyl)-amide

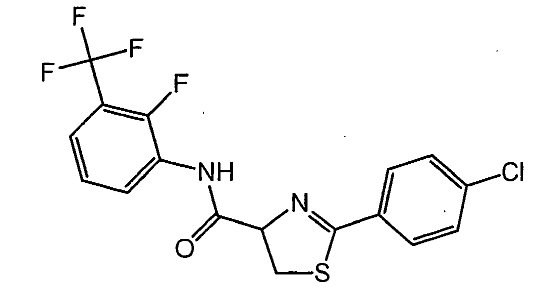

To a solution of 2-(4-chloro-phenyl)-4,5-dihydro-thiazole-4-carboxylic acid (112 mg, 0.463 mmol) and 3-amino-2-fluorobenzotrifluoride (91 mg, 0.509 mmol) in DMF (10 mL) were added DMAP (56.5 mg, .463 mmol), *i*Pr₂NEt (0.25 mL), and PyBroP (863 mg, 1.86 mmol) sequentially. The reaction mixture was stirred at room temperature overnight. The reaction mixture was diluted with water and extracted with EtOAc. The organic extracts were washed with 1N HCl and brine, then dried over anhydrous Na₂SO₄. The dessicant was filtered off and the filtrate was concentrated under vacuum to yield crude product. The crude product was purified by column chromatography (silica gel, 20% EtOAc/hexane) to yield 2-(4-chloro-phenyl)-4,5-dihydro-thiazole-4-carboxylic acid (2-fluoro-3-trifluoromethyl-phenyl)-amide.
¹H NMR (300 MHz, CDCl₃): δ 9.15 (br s, 1H), 8.64 (dt, 1H, J = 1.3, 7.0 Hz), 7.84 (d, 2H, J = 8.6 Hz), 7.79 - 7.89 (m, 1H), 7.45 (d, 2H, J = 8.6 Hz), 7.28 - 7.45 (m, 2H), 5.38 (t, 1H, 9.7 Hz), 3.73 - 3.89 (m, 2H)
MS m/z (M+H) = 403.

### Example 11 (Cmpd #14)

### 2-(4-chloro-phenyl)-4,5-dihydro-thiazole-4-carboxylic acid (4-fluoro-3-trifluoromethyl-phenyl)-amide

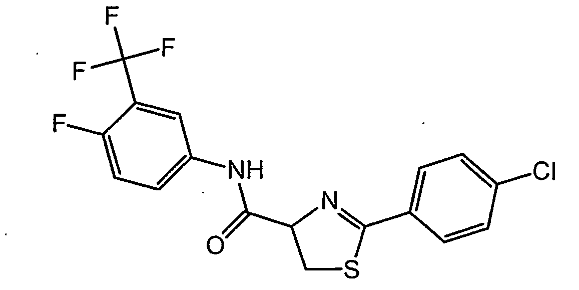

To a solution of 2-(4-chloro-phenyl)-4,5-dihydro-thiazole-4-carboxylic acid (112 mg, 0.4634 mmol) and 5-amino-2-fluorobenzotrifluoride (91.5 mg, 0.5107 mmol) in DMF (10 mL) were added DMAP (56.7mg, .4642 mmol), *i*Pr₂NEt (0.25 mL), and PyBroP (866 mg, 1.86 mmol) sequentially. The reaction mixture was stirred at room temperature overnight. The reaction mixture was diluted with water and extracted with EtOAc. The organic extracts were washed with 1N HCl and brine, then dried over anhydrous Na₂SO₄. The dessicant was filtered off and the filtrate was concentrated under vacuum to yield crude product. The crude product was purified by column chromatography (silica gel, 20% EtOAc/hexane) to yield 2-(4-chloro-phenyl)-4,5-dihydro-thiazole-4-carboxylic acid (4-fluoro-3-trifluoromethyl-phenyl)-amide.
¹H NMR (300 MHz, CDCl₃): δ 8.76 (br s, 1H), 7.84 (d, 2H, *J* = 8.6 Hz), 7.78 - 7.83 (m, 2H), 7.45 (d, 2H, *J* = 8.6 Hz), 7.18 (t, 1H, *J* = 9.5 Hz), 5.32 (t, 1H, *J* = 9.9 Hz), 3.71 - 3.88 (m, 2H)
MS m/z (M-H) = 401.

### Example 12 (Cmpd #25)

### 2-(4-fluoro-phenyl)-4,5-dihydro-thiazole-4-carboxylic acid (2-fluoro-3-trifluoromethyl-phenyl)-amide

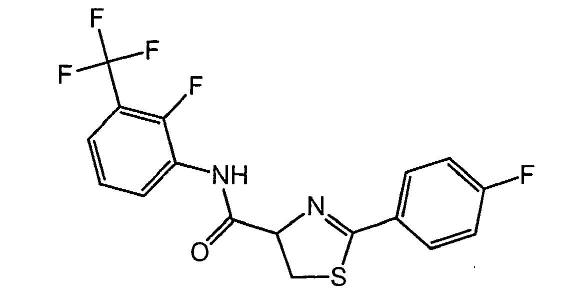

### Step A: fluoro-benzimidic acid ethyl ester hydrochloride

To a solution of 4-fluorobenzonitrile (7.89g, 65.2 mmol) in ethanol (20 mL) was bubbled in dry HCl (generated by adding 200 mL of 36% HCl dropwise into 500 mL of conc. H₂SO₄) at 5°C. The reaction mixture was stirred for an additional 0.5 hour at 0°C and then diluted with anhydrous diethyl ether (500 mL). After the mixture was stirred at 5°C for 2 hours, the solids were filtered, washed with diethyl ether, and dried under vacuum to yield fluoro-benzimidic acid ethyl ester hydrochloride. The product was carried on to the next step without further purification.

### Step B: 2-(4-fluoro-phenyl)-4,5-dihydro-thiazole-4-carboxylic acid methyl ester

Triethylamine (2.86g, 28:21 mmol) was added dropwise into a solution of fluoro-benzimidic acid ethyl ester hydrochloride (5.42 g, 26.62 mmol) and L-cysteine methyl ester hydrochloride (5.03g, 29.28 mmol) in dichloromethane (100 mL) at 0°C. The resulting mixture was stirred at room temperature for 3 days. Water and dichloromethane were added, the layers were separated, the aqueous layer was extracted with dichloromethane, dried over anhydrous Na₂SO₄, and concentrated under vacuum to give the crude product which was purified by column chromatography (silica gel, 15% EtOAc/hexane) to yield 2-(4-fluoro-phenyl)-4,5-dihydro-thiazole-4-carboxylic acid methyl ester.
¹H NMR (300 MHz,

### Step C: 2-(4-fluoro-phenyl)-4,5-dihydro-thiazole-4-carboxylic acid

To a solution of 2-(4-fluorophenyl)-4,5-dihydro-thiazole-4-carboxylic acid methyl ester (5.82g, 0.024 mole) in THF (200 mL) and H₂O (200 mL) was added KOH (13.65 g, 0.24 mole). The reaction mixture was vigorously stirred at room temperature for 17 hours. The reaction mixture was diluted with water, extracted with diethyl ether, and the diethyl ether layer was washed with water. The combined aqueous layers were acidified with 1N HCl to pH 1, extracted with diethyl ether, and dried over anhydrous Na₂SO₄. The dessicant was filtered off and the filtrate was concentrated under vacuum to yield 2-(4-fluorophenyl)-4,5-dihydro-thiazole-4-carboxylic acid.
¹H NMR (300 MHz, d₆-DMSO): δ 12.92 (br s, 1H), 7.83-7.88 (m, 2H), 731-7.37 (m, 2H), 5.30 (dd, 1H, J = =1.0, 8.4 Hz), 3.60-3.77 (m, 2H)

### Step D: 2-(4-fluoro-phenyl-4,5-dihydro-thiazole-4-carboxylic acid (2-fluoro-3-trifluoromethyl-phenyl)-amide

To a solution of 2-(4-fluoro-phenyl)-4,5-dihydro-thiazole-4-carboxylic acid (151 mg, 0.671 mmol) and 3-amino-2-fluorobenzotrifluoride (132 mg, 0.738 mmol) in DMF (10 mL) were added DMAP (82 mg, .671 mmol)), *i*Pr₂NEt (0.25 mL), and PyBroP (940 mg 2.01 mmol) sequentially. The reaction mixture was stirred at room temperature overnight. The reaction mixture was diluted with water and EtOAc. The organic layer was washed with 1N HCl and brine, then dried over anhydrous Na₂SO₄. The dessicant was filtered off and the filtrate was concentrated under vacuum to yield crude product. The crude product was purified by column chromatography (silica gel, 20% EtOAc/hexane) to yield 2-(4-fluoro-phenyl)-4,5-dihydro-thiazole-4-carboxylic acid (2-fluoro-3-trifluoromethyl-phenyl)-amide.
¹H NMR (300 MHz, CDCl₃): δ 9.17 (br s, 1H), 8.64 (dt, 1H, *J* = 1.4, 6.9 Hz), 7.87 - 7.94 (m, 3H), 7.07 - 7.36 (m, 4H), 5.37 (t, 1H, *J* = 9.6 Hz), 3.74 - 3.88 (m, 2H)
MS m/z (M-H) = 385.

### Example 13 (Cmpd #1)

### 2-(4-fluoro-phenyl)-4,5-dihydro-thiazole-4-carboxylic acid (3-trifluoromethyl-4-cyano-phenyl)-amide

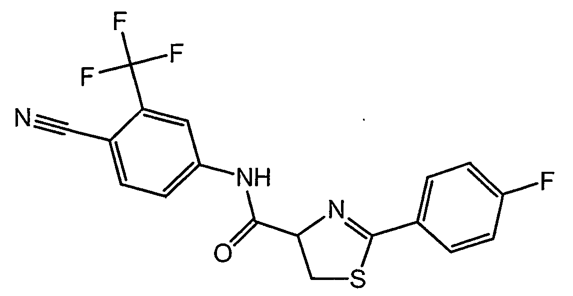

To a solution of 2-(4-fluoro-phenyl)-4,5-dihydro-thiazole-4-carboxylic acid methyl ester (201 mg, 0.840 mmol) and 5-amino2-cyanobenzotrifluoride (313 mg, 1.68 mmol) in THF (8 mL) at 0°C was added 2M isopropyl magnesium chloride (0.84 mL in THF). The reaction mixture was stirred at room temperature overnight. The reaction mixture was diluted with water and EtOAc. The organic layer was washed with 1N HCl and brine, then dried over anhydrous Na₂SO₄. The dessicant was filtered off and the filtrate was concentrated under vacuum to yield crude product. The crude product was purified by column chromatography (silica gel, 20% EtOAc/hexane) to yield 2-(4-fluoro-phenyl)-4,5-dihydro-thiazole-4-carboxyfic acid (4- cyano-3-trifluoromethyl-phenyl)-amide.
¹H NMR (400 MHz, CDCl₃): δ 9.06 (br s, 1H), 7.78 - 8.13 (m, 5H), 7.25 - 7.28 (m, 2H), 5.32 (t, 1H, *J* = 9.6 Hz), 3.71 - 3.88 (m, 2H)
MS m/z (M-H) = 392.

### Example 14 (Cmpd #7)

### 2-(4-fluoro-phenyl)-4,5-dihydro-thiazole-4-carboxylic acid (4-fluoro-3-trifluoromethyl-phenyl)-amide

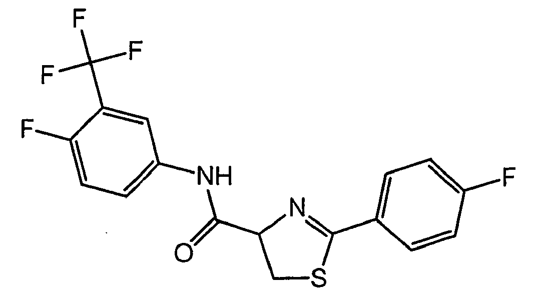

To a solution of 2-(4-fluoro-phenyl)-4,5-dihydro-thiazole-4-carboxylic acid (201 mg, 0.892 mmol) and 5-amino-2-fluorobenzotrifluoride (160 mg, 0.892 mmol) in DMF (10 mL) were added DMAP (109 mg, .892 mmol)), *i*Pr₂NEt (0.25 mL), and PyBroP (832 mg 1.785 mmol) sequentially. The reaction mixture was stirred at room temperature overnight. The reaction mixture was diluted with water and EtOAc. The organic layer was washed with 1 N HCI and brine, then dried over anhydrous Na₂SO₄. The dessicant was filtered off and the filtrate was concentrated under vacuum to give the crude product. The crude product was purified by column chromatography (silica gel, 20% EtOAc/hexane) to yield 2-(4-fluoro-phenyl)-4,5-dihydro-thiazole-4-carboxylic acid (4-fluoro-3-trifluoromethyl-phenyl)-amide.
MS m/z (M-H) = 385.

### Example 15 (Cmpd #6)

### 2-(4-fluoro-phenyl)-4,5-dihydro-thiazole-4-carboxylic acid (4-chloro-3-trifluoromethyl-phenyl)-amide

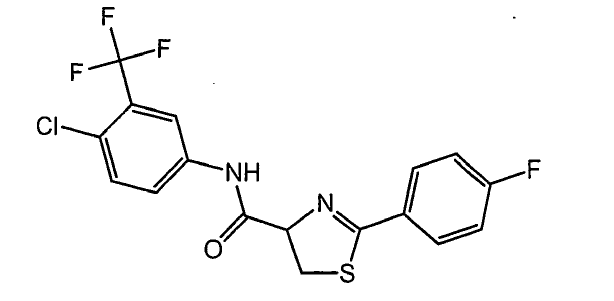

To a solution of 2-(4-fluoro-phenyl)-4,5-dihydro-thiazole-4-carboxylic acid (200 mg, 0.887 mmol) and 5-amino-2-chlorobenzotrifluoride (173 mg, 0.887 mmol) in DMF (10 mL) were added DMAP (108 mg, .887 mmol)), *i*Pr₂NEt (0.25 mL), and PyBroP (827 mg 1.773 mmol) sequentially. The reaction mixture was stirred at room temperature overnight. The reaction mixture was diluted with water and EtOAc. The organic layer was washed with 1N HCl and brine, then dried over anhydrous Na₂SO₄. The dessicant was filtered off and the filtrate was concentrated under vacuum to yield crude product. The crude product was purified by column chromatography (silica gel, 20% EtOAc/hexane) to yield 2-(4-fluoro-phenyl)-4,5-dihydro-thiazole-4-carboxylic acid (4-chloro-3-trifluoromethyl-phenyl)-amide.
¹H NMR (300 MHz, CDCl₃): δ 8.78 (br s, 1H), 7.79 - 8.03 (m, 4H), 7.13 - 7.21 (m, 3H), 5.31 (t, 1H, *J* = 9.8 Hz), 3.71 - 3.88 (m, 2H)
MS m/z (M-H) = 401.

### Example 16 (Cmpd #4)

### 2-(4-fluoro-phenyl)-4,5-dihydro-thiazole-4-carboxylic acid (4-nitro-3-trifluoromethyl-phenyl)-amide

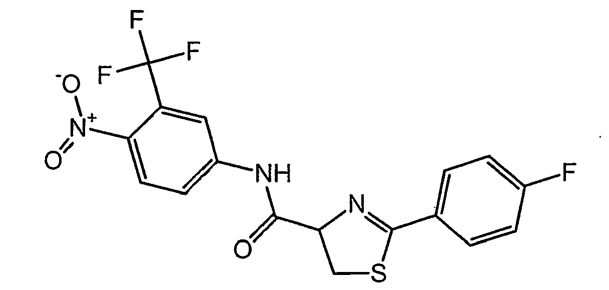

To a solution of 2-(4-fluoro-phenyl)-4,5-dihydro-thiazole-4-carboxylic acid (202 mg, 0.897 mmol) and 5-amino-2-nitrobenzotrifluoride (185 mg, 0.897 mmol) in DMF (10 mL) were added DMAP (110 mg, .897 mmol)), *i*Pr₂NEt (0.25 mL), and PyBroP (836 mg 1.794 mmol) sequentially. The reaction mixture was stirred at room temperature overnight. The reaction mixture was diluted with water and EtOAc. The organic layer was washed with 1N HCl and brine, then dried over anhydrous Na₂SO₄. The dessicant was filtered off and the filtrate was concentrated under vacuum to yield crude product. The crude product was purified by column chromatography (silica gel, 20% EtOAc/hexane) to yield 2-(4-fluoro-phenyl)-4,5-dihydro-thiazole-4-carboxylic acid (4-nitro-3-trifluoromethyl-phenyl)-amide.
¹H NMR (400 MHz, CDCl₃): δ 9.12 (br s, 1H), 7.79 - 8.08 (m, 5H), 7.15 - 7.20 (m, 2H), 5.34 (t, 1H, *J* = 10 Hz), 3.71 - 3.90 (m, 2H).
MS m/z (M-H) = 412.

### Example 17 (Cmpd #2)

### 2-(4-fluoro-phenyl)-4,5-dihydro-thiazole-4-carboxylic acid (4-cyano-phenyl)-amide

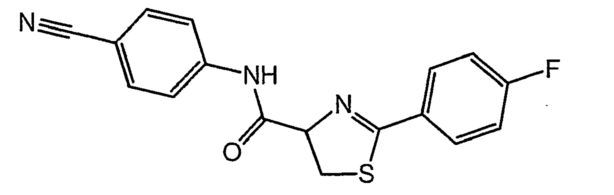

To a solution of 2-(4-fluoro-phenyl)-4,5-dihydro-thiazole-4-carboxylic acid methyl ester (250 mg, 1.045 mmol) and 4-aminobenzonitrile (247 mg, 2.089 mmol) in THF (10 mL) at 0°C was added 2M isopropyl magnesium chloride (1.04 mL in THF). The reaction mixture was stirred at room temperature overnight. The reaction mixture was diluted with water and EtOAc. The organic layer was washed with 1N HCl and brine, then dried over anhydrous Na₂SO₄. The dessicant was filtered off and the filtrate was concentrated under vacuum to yield crude product. The crude product was purified by column chromatography (silica gel, 20% EtOAc/hexane) to yield 2-(4-fluoro-phenyl)-4,5-dihydro-thiazole-4-carboxylic acid (4-cyano-phenyl)-amide.
¹H NMR (400 MHz, CDCl₃): δ 9.21 (br s, 1H), 7.63 - 7.52 (m, 4H), 7.08 - 7.19 (m, 4H), 5.32 (t, 1H, *J* = 9.6 Hz), 3.75 - 3.87 (m, 2H).
MS m/z (M-H) = 324.

### Example 18 (Cmpd #26)

### 2-(3-chloro-phenyl)-4,5-dihydro-thiazole-4-carboxylic acid (4-chloro-3-trifluoromethyl-phenyl)-amide

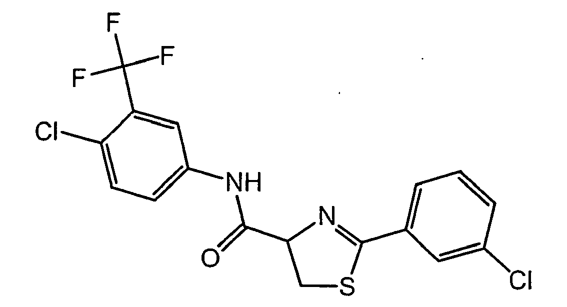

### Step A: 3-chloro-benzimidic acid ethyl ester hydrochloride

To a solution of 3-chlorobenzonitrile (11.36g, 85.6 mmol) in ethanol (30 mL) was bubbled in dry HCl (generated by adding 200 mL of 36% HCl dropwise into 500 mL of conc. H₂SO₄) at 5°C. The reaction mixture was stirred for an additional 0.5 hour at 5°C and then diluted with anhydrous diethyl ether (500 mL). After the mixture was allowed to sit at 5°C for 12 hours, the solids were filtered, washed with diethyl ether, and dried under vacuum to yield 3-chloro-benzimidic acid ethyl ester hydrochloride. The product was carried on to the next step without further purification.

### Step B: 2-(3-chloro-phenyl)-4,5-dihydro-thiazole-4-carboxylic acid methyl ester

Triethylamine (2.44g, 24.16 mmol) was added dropwise to a solution of 3-chloro-benzimidic acid ethyl ester hydrochloride (5 g, 22.71 mmol) and L-cysteine methyl ester hydrochloride (4.29 g, 24.98 mmol) in dichloromethane (85 mL) at 0°C. The resulting mixture was stirred at room temperature for 3 days. Water and dichloromethane were added, and the aqueous layer was extracted with dichloromethane, dried over anhydrous Na₂SO₄, and concentrated in vacuum to yield crude product which was purified by column chromatography (silica gel, 15% EtOAc/hexane) to yield 2-(3-chloro-phenyl)-4,5-dihydro-thiazole-4-carboxylic acid methyl ester.

### Step C: 2-(3-chloro-phenyl)-4,5-dihydro-thiazole-4-carboxylic acid

To a solution of 2-(3-chloro-phenyl)-4,5-dihydro-thiazole-4-carboxylic acid methyl ester (5 g, 0.0196 mole) in THF (200 mL) and H₂O (200 mL) was added KOH (10.97g, 0.196 mole). The reaction mixture was vigorously stirred at room temperature 17 hours. The reaction mixture was diluted with water, washed with diethyl ether and the organic layer was extracted with water. The combined aqueous layers were acidified with 1N HCl to pH 1, extracted with diethyl ether, and dried over anhydrous Na₂SO₄. The dessicant was filtered off and the filtrate was concentrated under vacuum to yield 2-(3-chloro-phenyl)-4,5-dihydro-thiazole-4-carboxylic acid.
¹H NMR (300MHz, d₆-DMSO): δ 13.07 (br s, 1H), 7.80 (t, 1H, *J* = 1.7 Hz), 7.73 (d, 1H, *J* = 7.7 Hz), 7.65 (t, 1H, *J* = 1.0 Hz), 7.55 (t, 1H, *J* = 7.8 Hz), 5.33 (dd, 1H, *J* = 8.3, 9.3 Hz), 3.64 - 3.78 (m, 2H).

### Step D: 2-(3-chloro-phenyl)-4,5-dihydro-thiazole-4-carboxylic acid (4-chloro-3-trifluoromethyl-phenyl)-amide

To a solution of 2-(3-chloro-phenyl)-4,5-dihydro-thiazole-4-carboxylic acid (147 mg, 0.606 mmol) and 5-amino-2-chlorobenzotrifluoride (130 mg, 0.667 mmol) in DMF (10 mL) were added DMAP (74 mg, .606 mmol)), *i*Pr₂NEt (0.25 mL), and PyBroP (848 mg 1.82 mmol) sequentially. The reaction mixture was stirred at room temperature overnight. The reaction mixture was diluted with water and EtOAc. The organic layer was washed with 1N HCl and brine, then dried over anhydrous Na₂SO₄. The dessicant was filtered off and the filtrate was concentrated under vacuum to yield crude product. The crude product was purified by column chromatography (silica gel, 20% EtOAc/hexane) to yield 2-(3-chloro-phenyl)-4,5-dihydro-thiazole-4-carboxylic acid (4-chloro-3-trifluoromethyl-phenyl)-amide.
¹H NMR (300 MHz, CDCl₃): δ 8.82 (br s, 1H), 7.90 - 7.95 (m, 2H), 7.72 - 7.80 (m, 2H), 7.37 - 7.52 (m, 3H), 5.32 (t, 1H, *J* = 10 Hz), 3.71 - 3.88 (m, 2H)
MS m/z (M-H) = 418.

### Example 19 (Cmpd #27)

### 2-(3-chloro-phenyl)-4,5-dihydro-thiazole-4-carboxylic acid (4-cyano-3-trifluoromethyl-phenyl)-amide

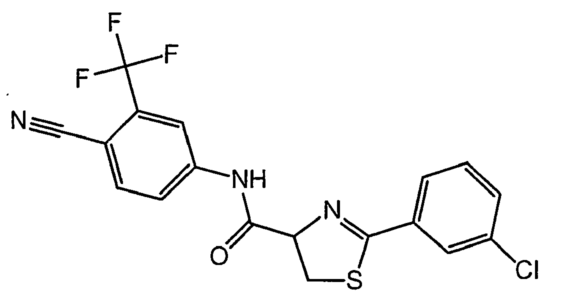

To a solution of 2-(3-chloro-phenyl)-4,5-dihydro-thiazole-4-carboxylic acid (162 mg, 0.6703 mmol) and 5-amino-2-cyanobenzotrifluoride (137 mg, 0.7373 mmol) in DMF (10 mL) were added DMAP (82 mg, .6703 mmol)), *i*Pr₂NEt (0.25 mL), and PyBroP (938 mg 2.01 mmol) sequentially. The reaction mixture was stirred at room temperature overnight. To the reaction mixture was added water and EtOAc. The organic layer was washed with 1N HCl and brine. The organic extracts were dried over anhydrous Na₂SO₄, filtered, and the filtrate was concentrated under vacuum to yield crude product. The crude product was purified by column chromatography (silica gel, 20% EtOAc/hexane) to yield 2-(3-chloro-phenyl)-4,5-dihydro-thiazole-4-carboxylic acid (4-cyano-3-trifluoromethyl-phenyl)-amide.
¹H NMR (300 MHz, CDCl₃): δ 9.05 (br s, 1H), 8.08 (d, 1H, *J* = 1.9 Hz), 7.98 (dd, 1H, *J* = 2.1, 8.5 Hz), 7.92 - 7.93 ((m, 1H), 7.82 (d, 1H, *J* = 8.5 Hz), 7.75 (dt, 1H, *J* = 1.3, 7.8 Hz), 7.52 (dd, 1H, *J* = 1.1, 2.0 Hz), 7.43 (t, 1H, *J* = 7.9 Hz), 5.35 (t, 1H, *J* = 10 Hz), 3.72 - 3.90 (m, 2H)
MS m/z (M-H) = 408.

### Example 20 (Cmpd #28)

### 2-(4-fluoro-phenyl)-4,5-dihydro-thiazole-4-carboxylic acid (4-trifluoromethanesulfonyl-phenyl)-amide

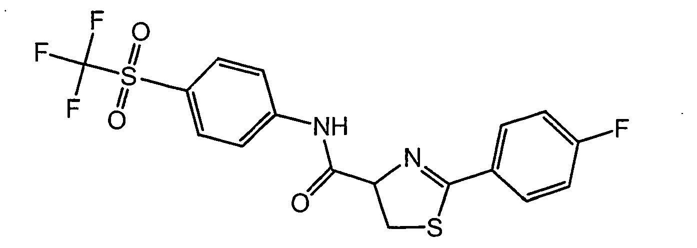

To a solution of 2-(4-fluoro-phenyl)-4,5-dihydro-thiazole-4-carboxylic acid (153 mg, 0.677 mmol) and 4-aminophenyl trifluoromethylsulfone (168 mg, 0.745 mmol) in DMF (10 mL) were added DMAP (83 mg, .6775 mmol)), *i*Pr₂NEt (0.25 mL), and PyBroP (948 mg 2.033 mmol) sequentially. The reaction mixture was stirred at room temperature overnight. To the reaction mixture was added water and EtOAc. The organic layer was washed with 1N HCl and brine. The organic extracts were dried over anhydrous Na₂SO₄, filtered, and the filtrate was concentrated under vacuum to yield crude product. The crude product was purified by column chromatography (silica gel, 20% EtOAc/hexane) to yield 2-(4-fluoro-phenyl)-4,5-dihydro-thiazole-4-carboxylic acid (4-trifluoromethanesulfonyl-phenyl)-amide.
¹H NMR (300 MHz, CDCl₃): δ 9.13 (br s, 1H), 7.88 - 8.01 (m, 6H), 7.12 - 7.20 (m, 2H), 5.35 (t, 1H, *J* = 10 Hz), 3.73 - 3.89 (m, 2H)
MS m/z (M-H) = 431.

### Example 21 (Cmpd #29)

### 2-(4-chloro-phenyl)-4,5-dihydro-thiazole-4-carboxylic acid (4-trifluoromethanesulfonyl-phenyl)-amide

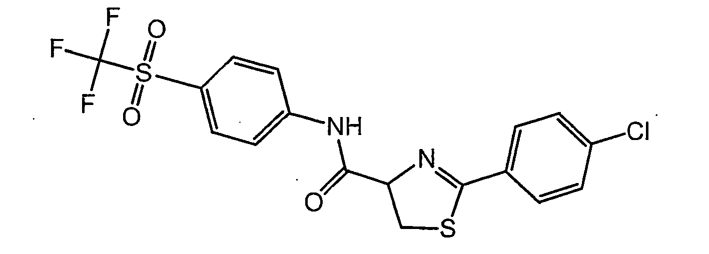

To a solution of 2-(4-chloro-phenyl)-4,5-dihydro-thiazole-4-carboxylic acid (172 mg, 0.711 mmol) and 4-aminophenyl trifluoromethylsulfone (176 mg, 0.7819 mmol) in DMF (10 mL) were added DMAP (87 mg, .7108 mmol)), *i*Pr₂NEt (0.25 mL), and PyBroP (994 mg 2.1324 mmol) sequentially. The reaction mixture was stirred at room temperature overnight. To the reaction mixture was added water and EtOAc. The organic layer was washed with 1N HCI and brine. The organic extracts were dried over anhydrous Na₂SO₄, filtered, and the filtrate was concentrated under vacuum to yeild crude product. The crude product was purified by column chromatography (silica gel, 20% EtOAc/hexane) to yield 2-(4-chloro-phenyl)-4,5-dihydro-thiazole-4-carboxylic acid (4-trifluoromethanesulfonyl-phenyl)-amide.
¹H NMR (300 MHz, CDCl₃): δ 9.08 (br s, 1H), 7.62 - 8.02 (m, 4H), 7.16 - 7.52 (m, 3H), 5.35 (t, 1H, J = 10 Hz), 3.74-3.86 (m, 2H)

### Example 22 (Cmpd #31)

### 2-(3-chloro-phenyl)-4,5-dihydro-thiazole-4-carboxylic acid (4-trifluoromethanesulfonyl-phenyl)-amide

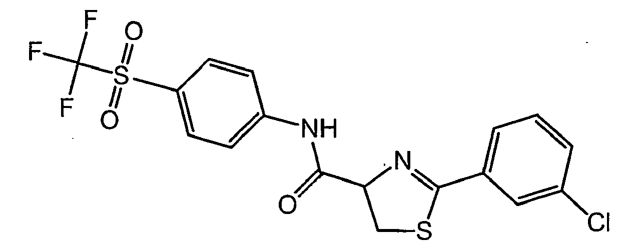

To a solution of 2-(3-chloro-phenyl)-4,5-dihydro-thiazole-4-carboxylic acid (170 mg, 0.701 mmol) and 4-aminophenyl trifluoromethylsulfone (174 mg, 0.771 mmol) in DMF (10 mL) were added DMAP (86 mg, 0.701 mmol)), *i*Pr₂NEt (0.25 mL), and PyBroP (981 mg 2.104 mmol) sequentially. The reaction mixture was stirred at room temperature overnight. To the reaction mixture was added water and EtOAc. The organic layer was washed with 1N HCl and brine. The organic extracts were dried over anhydrous Na₂SO₄, filtered, and the filtrate was concentrated under vacuum to yield crude product. The crude product was purified by column chromatography (silica gel, 20% EtOAc/hexane) to yield 2-(3-chloro-phenyl)-4,5-dihydro-thiazole-4-carboxylic acid (4-trifluoromethanesulfonyl-phenyl)-amide.
¹H NMR (300 MHz, CDCl₃): δ 9.09 (br s, 1H), 7.30 - 8.20 (m, 7 H), 5.36 (t, 1H, *J* = 9.9 Hz), 3.73 - 3.86 (m, 2 H)
MS m/z (M-H) = 447.

### Example 23 (Cmpd #30)

### 2-(3-chloro-phenyl)-4,5-dihydro-thiazole-4-carboxylic acid (4-nitro-3-trifluoromethyl-phenyl)-amide

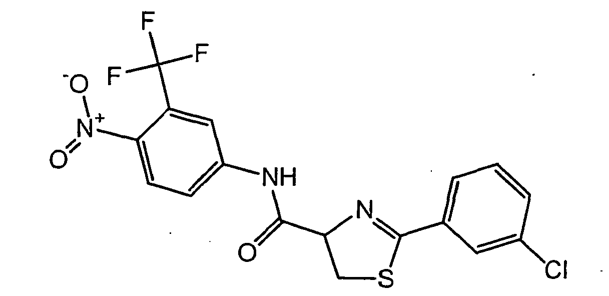

To a solution of 2-(3-chloro-phenyl)-4,5-dihydro-thiazole-4-carboxylic acid (169 mg, 0.701 mmol) and 5-amino-2-nitrobenzotrifluoride (159 mg, 0.771 mmol) in DMF (10 mL) were added DMAP (86 mg, .701 mmol)), *i*Pr₂NEt (0.25 mL), and PyBroP (980 mg 2.102 mmol) sequentially. The reaction mixture was stirred at room temperature overnight. To the reaction mixture was added water and EtOAc. The organic layer was washed with 1N HCl and brine. The organic extracts were dried over anhydrous Na₂SO₄, filtered, and the filtrate was concentrated under vacuum to yield crude product. The crude product was purified by column chromatography (silica gel, 20% EtOAc/hexane) to yield 2-(3-chloro-phenyl)-4,5-dihydro-thiazole-4-carboxylic acid (4-nitro-3-trifluoromethyl-phenyl)-amide.
MS m/z (M-H) = 428.

### Example 24 (Cmpd #32)

### 2-(3-chloro-phenyl)-4,5-dihydro-thiazole-4-carboxylic acid (2-fluoro-3-trifluoromethyl-phenyl)-amide

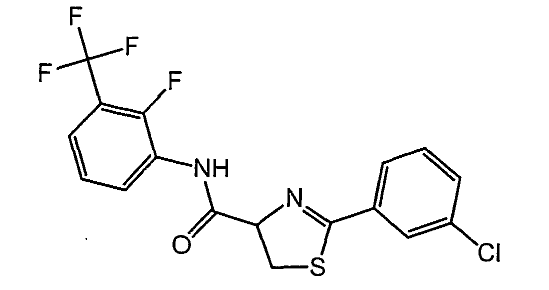

To a solution of 2-(3-chloro-phenyl)-4,5-dihydro-thiazole-4-carboxylic acid (167 mg, 0.691 mmol) and 3-amino-2-fluorobenzotrifluoride (136 mg, 0.760 mmol) in DMF (10 mL) were added DMAP (84 mg, 0.691 mmol)), *i*Pr₂NEt (0.25 mL), and PyBroP (966 mg 2.073 mmol) sequentially. The reaction mixture was stirred at room temperature overnight. To the reaction mixture was added water and EtOAc. The organic layer was washed with 1N HCl and brine. The organic extracts were dried over anhydrous Na₂SO₄, filtered, and the filtrate was concentrated under vacuum to yieldcrude product. The crude product was purified by column chromatography (silica gel, 20% EtOAc/hexane) to yield 2-(3-chloro-phenyl)-4,5-dihydro-thiazole-4-carboxylic acid (2-fluoro-3-trifluoromethyl-phenyl)-amide.
¹H NMR (300 MHz) □δ 9.13 (br s, 1H), 8.61 (dt, 1H, *J* = 1.3, 6.9), 7.77 - 7.88 (m, 2H), 7.27 - 7.53 (m, 4H), 5.39 (t, 1H, *J* = 9.7 Hz), 3.75 - 3.89 (m, 2H).

### Example 25 (Cmpd #33)

### 2-(4-fluoro-phenyl)-4-methyl-4,5-dihydro-thiazole-4-carboxylic acid (4-cyano-3-trifluoromethyl-phenyl)-amide

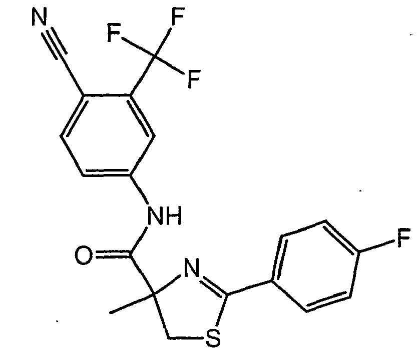

### Step A: 2-tert-butyl-3-formyl-4-methyl-thiazolidine-4-carboxylic acid ethyl ester

*n*-Butyllithium (2.5M in hexane 18 mL 0.0450 mole) was added dropwise to diisopropylamine (6.49g, 0.064 mole) in THF (225 mL) at -78°C. 1,3-dimethyl-tetrahydropyrimidin-2-one (32 mL) was then added add to the reaction mixture in one portion. The mixture reaction was stirred at -78°C for 1 hr. A solution of 2-*tert*-butyl-3-4-methyl-thiazolidine-4-carboxylic acid ethyl ester (10.5g, 0.0428 mole) in THF (20 mL) was added dropwise at-90°C. The solution was stirred at -90°C for 45 min. and then iodomethane (7.29 g, 0.0514 mole) was added. The resulting mixture was stirred at -90°C for 2 hours and then warmed to room temperature. The volatiles were removed under vacuum and the oily residue was partitioned between brine and diethyl ether. The aqueous layer was extracted with diethyl ether 3 times. The combined ethereal layer was dried over anhydrous Na₂SO₄. The dessicant was filtered and the filtrate was concentrated under vacuum to yield give crude product. The crude product was purified by column chromatography (silica gel, 20% EtOAc/hexane) to yield 2-*tert*-butyl-3-formyl-4-methyl-thiazolidine-4-carboxylic acid ethyl ester.
MS m/z (M+H) 282

### Step B: 2-amino-3-mercapto-2-methyl-propionic acid hydrochloride

2-*tert*-butyl-3-formyl-4-methyl-thiazolidine-4-carboxylic acid ethyl ester (1.34g, 5.17 mmol) and 5 N HCl were heated at 100°C for 15 hours. The mixture was then washed with EtOAc 3 times. The aqueous layer was evaporated to dryness to yield 2-amino-3-mercapto-2-methyl-propionic acid hydrochloride.
MS m/z (M+H) 136; (M-H) 134.

### Step C: 2-amino-3-mercapto-2-methyl-propionic acid methyl ester hydrochloride

Thionyl chloride (1.58g, 13.27 mmol) was added into a solution of 2-amino-3-mercapto-2-methyl-propionic acid hydrochloride (759 mg, 4.422 mmol) in CH₃OH (16 mL). The resulting mixture was heated at 65°C overnight. The volatiles were removed under vacuum to yield a foamy residue, which was dissolved in a small amount of CH₃OH and triturated with diethyl ether. A gummy compound precipitated out, and it was dried under vacuum to yield 2-amino-3-mercapto-2-methyl-propionic acid methyl ester hydrochloride. The crude compound was carried on to the next step without further purification.

### Step D: 2-(4-Fluoro-phenyl)-4-methyl-4, 5-dihydro-thiazole-4-carboxylic acid methyl ester

To a mixture of 4-fluorobenzimidic acid ethyl ester hydrochloride (5.42g, 0.794g, 0.0039 mole) and 2-amino-3-mercapto-2-methyl-propionic acid methyl ester hydrochloide (0.788 g, 0.004 mol) in dichloromethane (15 mL) at 0°C was added triethylamine (0.42 g, 0.004 mole) and subsequently stirred at room temperature for 2 days. Water and dichloromethane were added to the reaction mixture. The aqueous layer was extracted with dichloromethane. The organic layer was washed with brine and dried over anhydrous Na₂SO₄. The dessicant was filtered off and the filtrate was concentrated under vacuum to yield crude product. The crude product was purified by column chromatography (silica gel, 20% EtOAc/hexane) to yield 2-(4-fluoro-phenyl)-4-methyl-4, 5-dihydro-thiazole-4-carboxylic acid methyl ester as an oil.

### Step E: 2-(4-Fluoro-phenyl)-4-methyl-4, 5-dihydro-thiazole-4-carboxylic acid

To a solution of 2-(4-fluoro-phenyl)-4-methyl-4, 5-dihydro-thiazole-4-carboxylic acid methyl ester (5.82 g, 0.024 mole) in THF (200 mL) and water (200 mL) at room temperature was added KOH (13.65 g, 0.243 mole). The reaction was stirred at room temperature for 17 hours. The reaction mixture was diluted with water and extracted with diethyl ether. The aqueous layer was acidified with 50% HCl (aq) to pH 1, then extracted with diethyl ether. The diethyl ether layer was washed with brine and dried over anhydrous Na₂SO₄. The mixture was filtered and the filtrate was concentrated under vacuum to yield crude product. The crude product was purified by column chromatography (silica gel, 20% EtOAc/hexane) to yield 2-(4-fluoro-phenyl)-4-methyl-4, 5-dihydro-thiazole-4-carboxylic acid as an oil.

### Step F: 2-(4-fluoro-phenyl)-4-methyl-4, 5-dihydro-thiazole-4-carboxylic acid (4-cyano-3-trifluoromethyl-phenyl)-amide

To a solution of 2-(4-fluorophenyl)-4-methyl-4, 5-dihydro-thiazole-4-carboxylic acid (11 mg, 0.046 mmol) and 5-amino-2-cyanobenzotrifluoride (9.4 mg, 0.051 mmol) in DMF (1 mL) were added DMAP (5.6 mg, 0.046 mmol), *i*Pr₂NEt (0.25 mL), and PyBroP (64 mg, 0.138 mmol) sequentially. The reaction mixture was stirred at room temperature overnight. Water and EtOAc were added to the reaction mixture. The organic layer was washed with 1N HCl and brine, then dried over anhydrous Na₂SO₄. The dessicant was filtered off and the filtrate was concentrated under vacuum to yield crude product. The crude product was purified by column chromatography (silica gel, 20% EtOAc/hexane) to yield 2-(4-fluoro-phenyl)-4-methyl-4, 5-dihydro-thiazole-4-carboxylic acid (4-cyano-3-trifluoromethyl-phenyl)-amide.
MS m/z (M-H) 406.

Compound #12 was similarly prepared according to the procedure described in Example 25 above, substituting 4-chloro-3-trifluoromethyl-phenylamine for 5-amino-2-cyanobenzotrifluoride.

Compound #13 was similarly prepared according to the procedure described in Example 25 above, substituting 4-nitro-3-trifluoromethyl-phenylamine for 5-amino-2-cyanobenzotrifluoride.

### Example 26 (Cmpd #21)

### 2-(4-fluoro-phenyl)-5,5-dimethyl-4,5-dihydro-thiazole-4-carboxylic acid (4-chloro-3-trifluoromethyl-phenyl)-amide

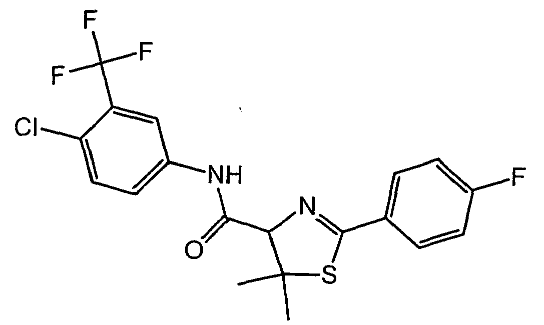

### Step A: 2-(4-fluoro-phenyl)-5,5-dimethyl-4,5-dihydro-thiazole-4-carboxylic acid methyl ester

Triethylamine (0.95 mls, 6.836 mmol) was added dropwise into a solution of 4-fluoro-benzimidic acid ethyl ester hydrochloride (1.33 g, 6.511 mmol) and penicillamine methyl ester hydrochloride (1.43 g, 7.163 mmol) in dichloromethane (20 mL) at 0°C. The resulting mixture was stirred at room temperature for 3 days. Water and dichloromethane were added, and the water layer was extracted with dichloromethane, dried over Na₂SO₄, and concentrated in vacuum to yield crude product. The crude product was purified by column chromatography (silica gel, 20 % EtOAc/hexane) to yield 2-(4-fluoro-5,5-dimethyl-phenyl)-4,5-dihydro-thiazole-4-carboxylic acid methyl ester.

### Step B: 2-(4-fluoro-phenyl)-5,5-dimethyl-4,5-dihydro-thiazole-4-carboxylic acid

To a solution of 2-(4-fluoro-phenyl)-5,5-dimethyl-4,5-dihydro-thiazole-4-carboxylic acid methyl ester (610 mg, 2.282 mmole) in THF (15 mL) and H₂O (5 mL) was added KOH (640 mg, 11.409 mmole). The mixture was vigorously stirred at room temperature overnight. The reaction mixture was diluted with water, washed with diethyl ether and the diethyl ether layer was extracted with water. The combined aqueous layers were acidified with 1N HCl to pH 1, then extracted with diethyl ether and dried over anhydrous Na₂SO₄. The dessicant was filtered off and the filtrate was concentrated under vacuum to yield 2-(4-fluoro-phenyl)-5,5-dimethyl-4,5-dihydro-thiazole-4-carboxylic acid.
¹H NMR (400 MHz, d₆-DMSO): δ 12.89 (br s, 1H), 7.78 - 7.83 (m, 2H), 7.28 - 7.34 (m, 2H), 4.85 (s, 1H), 1.71 (s, 3H), 1.43 (s, 3H).

### Step C: 2-(4-fluoro-phenyl)-5,5-dimethyl-4,5-dihydro-thiazole-4-carboxylic acid (4-chloro-3-trifluoromethyl-phenyl)-amide

To a solution of 2-(4-fluoro-phenyl)-5,5-dimethyl-4,5-dihydro-thiazole-4-carboxylic acid (162 mg, 0.638 mmol) and 5-amino-2-chlorobenzotrifluoride (137 mg, 0.701 mmol) in DMF (10 mL) were added DMAP (78 mg, .638 mmol)), *i*Pr₂NEt (0.25 mL), and PyBroP (892 mg 1.913 mmol) sequentially. The reaction mixture was stirred at room temperature overnight. The reaction mixture was diluted with water and EtOAc. The organic layer was washed with 1N HCl and brine, then dried over anhydrous Na₂SO₄. The dessicant was filtered off and the filtrate was concentrated under vacuum to yield crude product. The crude product was purified by column chromatography (silica gel, 20% EtOAc/hexane) to yield 2-(4-fluoro-phenyl)-5,5-dimethyl-4,5-dihydro-thiazole-4-carboxylic acid (4-chloro-3-trifluoromethyl-phenyl)-amide.
¹H NMR (400 MHz, CDCl₃): δ 9.30 (br s, 1H), 7.95 (d,1H, *J* = 2.5 Hz), 7.87 - 7.91 (m, 2H), 7.83 (dd, 1H, *J* = 2.5, 8.7 Hz), 7.45 (d, 1H, *J* = 8.7 Hz), 7.13-7.17 (m, 2H), 4.73 (s, 1H), 1.96 (s, 3H), 1.47 (s, 3H)
MS m/z (M-H) = 429.

### Example 27 (Cmpd #19)

### 2-(4-fluoro-phenyl)-5,5-dimethyl-4,5-dihydro-thiazole-4-carboxylic acid (4-cyano-3-trifluoromethyl-phenyl)-amide

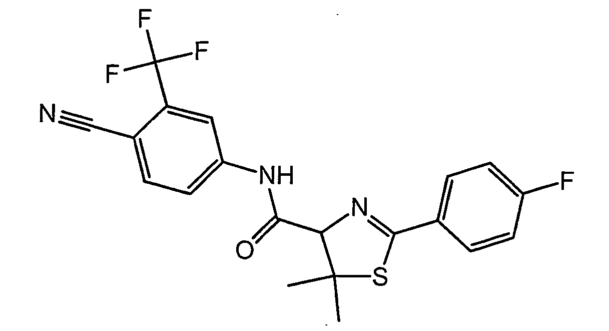

To a solution of 2-(4-fluoro-phenyl)-5,5-dimethyl-4,5-dihydro-thiazole-4-carboxylic acid (180 mg, 0.709 mmol) and 5-amino-2-cyanobenzotrifluoride (145 mg, 0.779 mmol) in DMF (10 mL) were added DMAP (87 mg, 0.709 mmol)), *i*Pr₂NEt (0.25 mL), and PyBroP (991 mg, 2.126 mmol) sequentially. The reaction mixture was stirred at room temperature overnight. The reaction mixture was diluted with water and EtOAc. The organic layer was washed with 1N HCl and brine, then dried over anhydrous Na₂SO₄. The dessicant was filtered off and the filtrate was concentrated under vacuum to yield crude product. The crude product was purified by column chromatography (silica gel, 20% EtOAc/hexane) to yield 2-(4-fluoro-phenyl)-5,5-dimethyl-4,5-dihydro-thiazole-4-carboxylic acid (4-cyano-3-trifluoromethyl-phenyl)-amide.
¹H NMR (300 MHz, CDCl₃): δ 9.57 (br s, 1H), 8.09 (d, 1H, *J* = 2.0 Hz), 8.01 (dd, 1H, *J* = 2.1, 8.5 Hz), 7.87 - 7.92 (m, 2H), 7.81 (d, 1H, *J* = 8.5 Hz), 7.13 - 7.20 (m, 2H), 4.75 (s, 1H), 1.96 (s, 3H), 1.47 (s, 3H).
MS m/z (M-H) = 420.

### Example 28 (Cmpd #20)

### 2-(4-fluoro-phenyl)-5,5-dimethyl-4,5-dihydro-thiazole-4-carboxylic acid (4-nitro-3-trifluoromethyl-phenyl)-amide

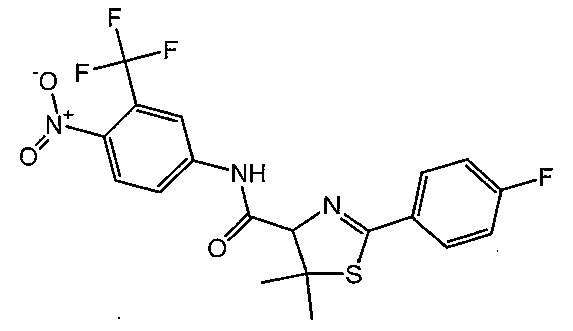

To a solution of 2-(4-fluoro-phenyl)-5,5-dimethyl-4,5-dihydro-thiazole-4-carboxylic acid (181 mg, 0.714 mmol) and 5-amino-2-nitrobenzotrifluoride (162 mg, 0.785 mmol) in DMF (10 mL) were added DMAP (87 mg, 0.709 mmol)), *i*Pr₂NEt (0.25 mL), and PyBroP (998 mg, 2.141 mmol) sequentially. The reaction mixture was stirred at room temperature overnight. The reaction mixture was diluted with water and EtOAc. The organic layer was washed with 1N HCl and brine, then dried over anhydrous Na₂SO₄. The dessicant was filtered off and the filtrate was concentrated under vacuum to yield crude product. The crude product was purified by column chromatography (silica gel, 20% EtOAc/hexane) to yield 2-(4-fluoro-phenyl)-5,5-dimethyl-4,5-dihydro-thiazole-4-carboxylic acid (4-nitro-3-trifluoromethyl-phenyl)-amide.
¹H NMR (300 MHz, CDCl₃): δ 9.60 (br s, 1H), 8.06 - 8.10 (m, 2H), 7.98 - 8.01 (m, 1H), 7.88 - 7.92 (m, 2H), 7.14 - 7.20 (m, 2H), 4.77 (s, 1H), 1.97 (s, 3H), 1.47 (s, 3H)
MS m/z (M-H) = 440.

### Example 29 (Cmpd#22)

### 2-(4-chloro-phenyl)-5,5-dimethyl-4,5-dihydro-thiazole-4-carboxylic acid (4-chloro-3-trifluoromethyl-phenyl)-amide

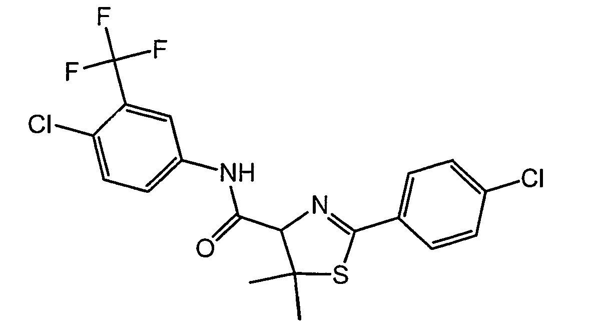

### Step A: 2-(4-chloro-phenyl)-5,5-dimethyl-4,5-dihydro-thiazole-4-carboxylic acid methyl ester

Triethylamine (2 mls, 14.31 mmol) was added dropwise into a solution of 4-chloro-benzimidic acid ethyl ester hydrochloride (3.0 g, 13.630 mmol) and penicillamine methyl ester hydrochloride (3.0 g, 14.990 mmol) in dichloromethane (50 mL) at 0°C. The resulting mixture was stirred at room temperature for 3 days. Water and dichloromethane was added, and the water layer was extracted with dichloromethane, dried over Na₂SO₄, and concentrated in vacuum to yield crude product. The crude product was purified by column chromatography (silica gel, 15 % EtOAc/hexane) to yield 2-(4-chloro-5,5-dimethyl-phenyl)-4,5-dihydro-thiazole-4-carboxylic acid methyl ester.

### Step B: 2-(4-chloro-phenyl)-5,5-dimethyl-4,5-dihydro-thiazole-4-carboxylic acid

To a solution of 2-(4-chloro-phenyl)-5,5-dimethyl-4,5-dihydro-thiazole-4-carboxylic acid methyl ester (1.4 g, 4.930 mmole) in THF (30 mL) and H₂O (10 mL) was added KOH (1.38 g, 24.660 mmole). The mixture was vigorously stirred at room temperature overnight. The reaction mixture was diluted with water, washed with diethyl ether and the diethyl ether layer was extracted with water. The combined aqueous layers were acidified with 1N HCl to pH 1, then extracted with diethyl ether and dried over anhydrous Na₂SO₄. The dessicant was filtered off and the filtrate was concentrated under vacuum to yield 2-(4-chloro-phenyl)-5,5-dimethyl-4,5-dihydro-thiazole-4-carboxylic acid.
¹H NMR (400 MHz, d₆-DMSO): δ 12.85 (br s, 1H), 7.76 (d, 2H, *J* = 8.6 Hz), 7.56 (d, 2H, *J* = 8.6 Hz), 4.87 (s, 1H), 1.71 (s, 3H), 1.43 (s, 3H).

### Step C: 2-(4-chloro-phenyl)-5,5-dimethyl-4,5-dihydro-thiazole-4-carboxylic acid (4-chloro-3-trifluoromethyl-phenyl)-amide

To a solution of 2-(4-chloro-phenyl)-5,5-dimethyl-4,5-dihydro-thiazole-4-carboxylic acid (162 mg, 0.601 mmol) and 5-amino-2-chlorobenzotrifluoride (129 mg, 0.661 mmol) in DMF (10 mL) were added DMAP (73 mg, 0.601 mmol)), *i*Pr₂NEt (0.25 mL), and PyBroP (841 mg, 1.803 mmol) sequentially. The reaction mixture was stirred at room temperature overnight. The reaction mixture was diluted with water and EtOAc. The organic layer was washed with 1N HCl and brine, then dried over anhydrous Na₂SO₄. The dessicant was filtered off and the filtrate was concentrated under vacuum to yield crude product. The crude product was purified by column chromatography (silica gel, 20% EtOAc/hexane) to yield 2-(4-chloro-phenyl)-5,5-dimethyl-4,5-dihydro-thiazole-4-carboxylic acid (4-chloro-3-trifluoromethyl-phenyl)-amide.
¹H NMR (300 MHz, CDCl₃): δ 9.28 (br s, 1H), 7.95 (d, 1H, *J* = 2.5 Hz), 7.80- 7.85 (m, 3H), 7.42 - 7.49 (m, 3H), 4.73 (s, 1H), 1.96 (s, 3H), 1.47 (s, 3H).

### Example 30 (Cmpd #23)

### 2-(4-chloro phenyl)-5,5-dimethyl-4,5-dihydro-thiazole-4-carboxylic acid (4-cyano-2,5-difluoro-ahenyl)-amide

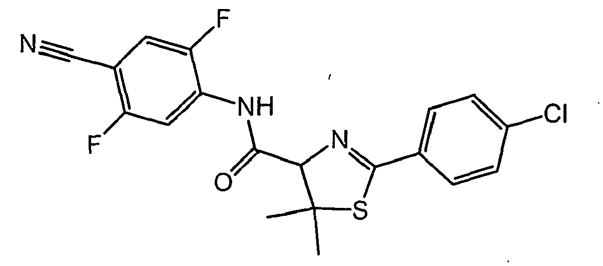

To a solution of 2-(4-chloro-phenyl)-5,5-dimethyl-4,5-dihydro-thiazole-4-carboxylic acid (162 mg, 0.601 mmol) and 4-amino-2,5-difluorobenzonitrile (131 mg, 0.662 mmol) in DMF (10 mL) were added DMAP (74 mg, 0.602 mmol)), *i*Pr₂NEt (0.25 mL), and PyBroP (842 mg, 1.806 mmol) sequentially. The reaction mixture was stirred at room temperature overnight. The reaction mixture was diluted with water and EtOAc. The organic layer was washed with 1N HCl and brine, then dried over anhydrous Na₂SO₄. The dessicant was filtered off and the filtrate was concentrated under vacuum to yield crude product. The crude product was purified by column chromatography (silica gel, 17 % EtOAc/hexane) to yield 2-(4-chloro-phenyl)-5,5-dimethyl-4,5-dihydro-thiazole-4-carboxylic acid (4-cyano-2,5-difluoro-phenyl)-amide.
¹H NMR (300 MHz, CDCl₃): δ 9.28 (br s, 1H), 7.95 (d, 1H, *J* = 2.5 Hz), 7.80 - 7.85 (m, 3H), 7.42 - 7.49 (m, 3H), 4.73 (s, 1H), 1.96 (s, 3H), 1.47 (s, 3H).

### Example 31

### In Vitro Assay - Androgen Receptor Filtration Binding Assay

The assay was run on a 96 well plate with each well filled with total reaction volume 150 µL, of a solution containing 5 pmol androgen receptor LBD (Panvera) or 30µL of freshly prepared rat cytosol, 0.5 nM [³H] R1881 tracer (NEN), 1.5 µL (10 µM) test compound or vehicle (diluted in 30% DMSO, final concentration of DMSO 0.75%) and 150 µL of TED buffer. (TED buffer contains 10 mM Tris.HCl pH 7.4, 1 mM sodium molybdate (60 mg/250 mL), 1.5 mM EDTA, 1 mM DTT and 10% (v/v) glycerol.)

On day one, the solution containing receptor, tracer and TED buffer was distributed onto a 96 well plate. Diluted test compound or control vehicle was then added to individual wells and the plate incubated at 4°C overnight.

On day two, to each well was then added 20 µL human γ-globulin (ICN 823102), prepared at 25 mg/ml in TE pH 8.0, and 55 µL 40% polyethylene glycol 8000 (JT Baker U222-08), prepared in TE pH 8.0. The plate was incubated at 4°C for 60 minutes. During incubation, the harvester was rinsed with 10% PEG 8000, prepared in TE pH 8.0 and the GF/C Unifilter-96 prewet with 10% PEG. The binding reaction was filtered, the retentate was washed three times with 10% PEG and dried under vacuum for about four minutes, then dried at 50°C for 5 min and then bottom sealed. 25 µL of Microscint-20 (Packard) was added to the filter wells and top sealed. The plate wells were then counted on a TopCount (Packard).

Selected compounds of the present invention were tested for binding to the androgen receptor according to the procedure described above with results as listed in Table 2. Negative number(s) indicate that there is no antagonist activity for the test compound. The compound may however have agonist activity, which was not tested for.

**TABLE 2 - Androgen Receptor Binding Results**

| **Cmpd #** | **% Inhibition** | **lC₅₀** |
|---|---|---|
| 1 | 50.0 @ 10µM | |
| | 28.5 @ 1µM | |
| 2 | -26.0 @ 1µM | |
| 3 | 8.1 @ 1µM | |
| 4 (†) | 50.0 @ 1µM | |
| | -24.0 @ 1µM | |
| 5 | 12.3 @ 1µM, | |
| | (average of 2) | |
| 7 | -24.25 @ 1µM | |
| | (average of 2) | |
| 9 | 41.5 @ 1µM | |
| 10 | 60.5 @ 1µM | |
| 11 | 30.5 @ 1µM | 3500 nM |
| 12 | 52.0 @ 1µM | >10,000 nM |
| 13 | 29.0 @ 1µM | >10,000 nM |
| 14 | 1.5 @ 1µM | |
| 16 | 12.0 @ 1µM | |
| 17 | -34.0 @ 1µM | |
| 18 | -19.0 @ 1µM | |
| 19 | -1.0 @ 1µM | |
| 20 | 29.0 @ 1µM | |
| 21 | 29.0 @ 1µM | |
| 22 | 0 @ 1µM | |
| 23 | -5.0 @ 1µM | |
| 24 | 0.74 @ 1µM | |
| 25 | -8.0 @ 1µM | |
| 26 | 15.0 @ 1µM | |
| 27 | 34.0 @ 1µM | |
| 28 | -6.3 @ 1µM | |
| 29 | 25.0 @ 1µM | |
| 30 | 7.0 @ 1µM | |
| 31 | 10.0 @ 1µM | |
| 32 | 8.5 @ 1 µM | |
| 33 | 27.0 @ 1 µM | |

| | | |
|---|---|---|
| † Results for cmpd. #4 are based on two separate synthesis batches. A confirmatory third batch was not as of the time of filing. * Negative values indicate no antagonist activity. Compounds with no measured antagonist activity may possess agonist activity. | | |

While the foregoing specification teaches the principles of the present invention, with examples provided for the purpose of illustration, it will be understood that the practice of the invention encompasses all of the usual variations, adaptations and/or modifications as come within the scope of the following claims and their equivalents.

## Claims

1. A compound of formula (I) 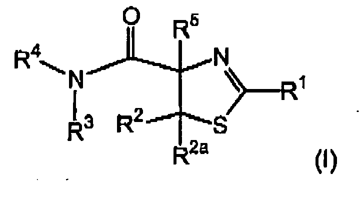 wherein
R¹ is selected from the group consisting of aryl and heteroaryl;
wherein the heteroaryl group is bound through a carbon atom;
wherein the aryl or heteroaryl group is optionally substituted with one to four substituent independently selected from halogen, alkyl, halogen substituted alkyl, C₁₋₄ alkyl ester, cyano, N(R^{A})₂C(O)-, C₁₋₄ alkyl-C(O)-NR^{A}-, C₁₋₄ alkyl-S(O)₀₋₂-, phenyl-S(O)₀₋₂, C₁₋₄ alkyl-S(O)₀₋₂-NR^{A}-, phenyl-S(O)₀₋₂-NR^{A}- and trifluoromethy-sulfonyl,
wherein the phenyl is optionally substituted with one or more substitutents independently selected from halogen, C₁₋₄ alkyl, C₁₋₄ alkoxy, hydroxy, carboxy, cyano, nitro, amino, C₁₋₄ alkylamino or di(C₁₋₄ alkyl)amino;
R^{A} is independently selected from hydrogen or C₁₋₄ alkyl;
R² and R^{2a} are each independently selected from the group consisting of hydrogen, C₁₋₄ alkyl and halogen substituted C₁₋₄ alkyl;
R³ is selected from the group consisting of hydrogen and C₁₋₄ alkyl;
R⁴ is selected from the group consisting of aryl and heteroaryl;
wherein the heteroaryl group is bound through a carbon atom;
wherein the aryl or heteroaryl group is optionally substituted with one to four substituent independently selected from halogen, alkyl, halogen substituted alkyl, C₁₋₄ alkyl ester, cyano, N(R^{A})₂C(O)-, C₁₋₄ alkyl-C(O)-NR^{A}-, C₁₋₄ alkyl-S(O)₀₋₂-, phenyl-S(O)₀₋₂, C₁₋₄ alkyl-S(O)₀₋₂-NR^{A}-, phenyl-S(O)₀₋₂-NR^{A}- and trifluoromethyl-sulfonyl;
wherein the phenyl is optionally substituted with one or more substitutents independently selected from halogen, C₁₋₄ alkyl, C₁₋₄ alkoxy, hydroxy, carboxy, cyano, nitro, amino, C₁₋₄ alkylamino or di(C₁₋₄ alkyl)amino;
R⁶ is selected from the group consisting of hydrogen, C₁₋₄ alkyl and halogen substituted C₁₋₄ alkyl;
or a pharmaceutically acceptable salt thereof.

2. A compound as in Claim 1 wherein
R¹ is selected from the group consisting of aryl;
wherein the aryl group is optionally substituted with one to two substituent independently selected from halogen, alkyl, halogen substituted alkyl, C₁₋₄ alkyl ester, cyano, N(R^{A})₂C(O)-, C₁₋₄ alkyl-C(O)-NR^{A}-, C₁₋₄ alkyl-S(O)₀₋₂-, phenyl-S(O)₀₋₂, C₁₋₄ alkyl-S(O)₀₋₂-NR^{A}-, phenyl-S(O)₀₋₂-NR^{A}- and trifluoromethyl-sulfonyl;
wherein the phenyl is optionally substituted with one or more substitutents independently selected from halogen, C₁₋₄ alkyl, C₁₋₄ alkoxy, hydroxy, carboxy, cyano, nitro, amino, C₁₋₄ alkylamino or di(C₁₋₄ alkyl)amino;
R² is selected from the group consisting of hydrogen, C₁₋₄ alkyl and trifluoromethyl;
R^{2a} is selected from the group consisting of hydrogen, C₁₋₄ alkyl and trifluoromethyl;
R³ is selected from the group consisting of hydrogen and C₁₋₄ alkyl;
R⁴ is selected from the group consisting of aryl;
wherein the aryl group is optionally substituted with one to two substituent independently selected from halogen, alkyl, halogen substituted alkyl, C₁₋₄ alkyl ester, cyano, N(R^{A})₂C(O)-, C₁₋₄ alkyl-C(O)-NR^{A}-, C₁₋₄ alkyl-S(O)₀₋₂-, phenyl-S(O)₀₋₂, C₁₋₄ alkyl-S(O)₀₋₂-NR^{A}-, phenyl-S(O)₀₋₂-NR^{A}- and trifluoromethyl-sulfonyl;
wherein the phenyl is optionally substituted with one or more substituents independently selected from halogen, C₁₋₄ alkyl, C₁₋₄ alkoxy, hydroxy, carboxy, cyano, nitro, amino, C₁₋₄ alkylamino or di(C₁₋₄ alkyl)amino;
R⁵ is selected from the group consisting of hydrogen, C₁₋₄ alkyl and trifluoromethyl;
or a pharmaceutically acceptable salt thereof.

3. A compound as in Claim 2 wherein
R¹ is selected from the group consisting of aryl; wherein the aryl group is optionally substituted with a halogen;
R² is selected from the group consisting of hydrogen and C₁₋₄ alkyl;
R^{2a} is selected from the group consisting of hydrogen and C₁₋₄ alkyl;
R³ is hydrogen;
R⁴ is selected from the group consisting of aryl, wherein the aryl group is substituted with one to three substituents independently selected from halogen, cyano, nitro, C₁₋₄ alkyl, halogen substituted C₁₋₄ alkyl and trifluoromethyl-sulfonyl;
R⁵ is selected from the group consisting of hydrogen and C₁₋₄ alkyl;
or a pharmaceutically acceptable salt thereof.

4. A compound as in Claim 3 wherein
R¹ is selected from the group consisting of 3-fluorophenyl, 4-fluorophenyl, 3-chlorophenyl and 4-chlorophenyl;
R² is selected from the group consisting of hydrogen and methyl;
R^{2a} is selected from the group consisting of hydrogen and methyl;
R³ is hydrogen;
R⁴ is selected from the group consisting of 4-chlorophenyl, 4-cyanaphenyl, 3-trifluoromethyl-4-cyano-phenyl, 3-trifluoromethyl-4-nitro-phenyl, 3-trifluoromethyl-4-chloro-phenyl, 3-trifluoromethyl-4-fluoro-phenyl, 2,5-difuoro-4-cyano-phenyl, 2-fluoro-3-trifluoromethyl-phenyl and 4-trifluoromethyl-sulfonyl-phenyl;
R⁵ is selected from the group consisting of hydrogen and methyl;
or a pharmaceutically acceptable salt thereof.

5. A compound as in Claim 4 wherein
R¹ is selected from the group consisting of 4-fluorophenyl, 3-chlorophenyl and 4-chlorophenyl;
R² is selected from the group consisting of hydrogen and methyl;
R^{2a} is selected from the group consisting of hydrogen and methyl;
R³ is hydrogen;
R⁴ is selected from the group consisting of 3-trifluoromethyl-4-cyano-phenyl, 3-trifluoromethyl-4-nitro-phenyl, 3-trifluoromethyl-4-chloro-phenyl and 4-trifluoromethylsulfonyl-phenyl;
R⁶ is selected from the group consisting of hydrogen and methyl;
or a pharmaceutically acceptable salt thereof.

6. A compound as in Claim 5 wherein
R¹ is selected from the group consisting of 4-fluorophenyl and 4-chlorophenyl;
R² is hydrogen;
R^{2a} is hydrogen;
R³ is hydrogen;
R⁴ is selected from the group consisting of 3-trifluoromethyl-4-nitro-phenyl and 3-trifluoromethyl-4-chloro-phenyl;
R⁶ is selected from the group consisting of hydrogen and methyl;
or a pharmaceutically acceptable salt thereof.

7. A pharmaceutical composition comprising a pharmaceutically acceptable carrier and a compound of Claim 1.

8. A pharmaceutical composition made by mixing a compound of Claim 1 and a pharmaceutically acceptable carrier.

9. A process for making a pharmaceutical composition comprising mixing a compound of Claim 1 and a pharmaceutically acceptable carrier.

10. A compound according to any of claims 1 to 6 for use in medicine.

11. Use of the compound of Claim 1 in the manufacture of a medicament for treating a disorder mediated by an androgen receptor, in a subject in need thereof.

12. Use of the compound of Claim 1 in the manufacture of a medicament for treating a condition selected from the group consisting of prostate carcinoma, benign prostatic hyperplasia (BPH), hirsutism alopecia, anorexia nervosa, breast cancer, acne, AIDS, cachexia, male contraception, and male performance, in a subject in need thereof.

## Patentansprüche

1. Verbindung von Formel (I) 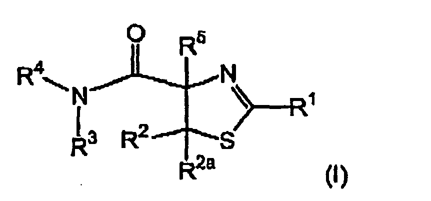 worin
R¹ ausgewählt ist aus der Gruppe, bestehend aus Aryl und Heteroaryl;
wobei die Heteroarylgruppe durch ein Kohlenstoffatom gebunden ist;
wobei die Aryl- oder Heteroarylgruppe fakultativ mit einem bis vier Substituenten substituiert ist, die unabhängig ausgewählt sind aus Halogen, Alkyl, halogensubstituiertem Alkyl, C₁₋₄-Alkylester, Cyano, N(R^{A})₂C(O)-, C₁₋₄-Alkyl-C(O)-NR^{A}-, C₁₋₄-Alkyl-S(O)₀₋₂-, Phenyl-S(O)₀₋₂, C₁₋₄-Alkyl-S(O)₀₋₂-NR^{A}-, Phenyl-S(O)₀₋₂-NR^{A}- und Trifluormethylsulfonyl;
wobei das Phenyl fakultativ mit einem oder mehreren Substituenten substituiert ist, die unabhängig ausgewählt sind aus Halogen, C₁₋₄-Alkyl, C₁₋₄-Alkoxy, Hydroxy, Carboxy, Cyano, Nitro, Amino, C₁₋₄-Alkylamino oder Di(C₁₋₄-alkyl)amino;
R^{A} unabhängig ausgewählt ist aus Wasserstoff oder C₁₋₄-Alkyl;
R² und R^{2a} jeweils unabhängig ausgewählt sind aus der Gruppe, bestehend aus Wasserstoff, C₁₋₄-Alkyl und halogensubstituiertem C₁₋₄-Alkyl;
R³ ausgewählt ist aus der Gruppe, bestehend aus Wasserstoff und C₁₋₄-Alkyl;
R⁴ ausgewählt ist aus der Gruppe, bestehend aus Aryl und Heteroaryl;
wobei die Heteroarylgruppe durch ein Kohlenstoffatom gebunden ist;
wobei die Aryl- oder Heteroarylgruppe fakultativ mit einem bis vier Substituenten substituiert ist, die unabhängig ausgewählt sind aus Halogen, Alkyl, halogensubstituiertem Alkyl, C₁₋₄-Alkylester, Cyano, N(R^{A})₂C(O)-, C₁₋₄-Alkyl-C(O)-NR^{A}-, C₁₋₄-Alkyl-S(O)₀₋₂-, Phenyl-S(O)₀₋₂, C₁₋₄-Alkyl-S(O)₀₋₂-NR^{A}-, Phenyl-S(O)₀₋₂-NR^{A}- und Trifluormethylsulfonyl;
wobei das Phenyl fakultativ substituiert ist mit einem oder mehreren Substituenten, die unabhängig ausgewählt sind aus Halogen, C₁₋₄-Alkyl, C₁₋₄-Alkoxy, Hydroxy, Carboxy, Cyano, Nitro, Amino, C₁₋₄-Alkylamino oder Di(C₁₋₄-alkyl)amino;
R⁵ ausgewählt ist aus der Gruppe, bestehend aus Wasserstoff, C₁₋₄-Alkyl und halogensubstituiertem C₁₋₄-Alkyl;
oder ein pharmazeutisch annehmbares Salz davon.

2. Verbindung nach Anspruch 1, **dadurch gekennzeichnet, daß**
R¹ ausgewählt ist aus der Gruppe, bestehend aus Aryl;
wobei die Arylgruppe fakultativ mit einem bis zwei Substituenten substituiert ist, die unabhängig ausgewählt sind aus Halogen, Alkyl, halogensubstituiertem Alkyl, C₁₋₄-Alkylester, Cyano, N(R^{A})₂C(O)-, C₁₋₄Alkyl-C(O)-NR^{A}-, C₁₋₄-Alkyl-S(O)₀₋₂-, Phenyl-S(O)₀₋₂, C₁₋₄-Alkyl-S(O)₀₋₂-NR^{A}-, Phenyl-S(O)₀₋₂-NR^{A}- und Trifluormethylsulfonyl;
wobei das Phenyl fakultativ substituiert ist mit einem oder mehreren Substituenten, die unabhängig ausgewählt sind aus Halogen, C₁₋₄-Alkyl, C₁₋₄-Alkoxy, Hydroxy, Carboxy, Cyano, Nitro, Amino, C₁₋₄-Alkylamino oder Di(C₁₋₄-alkyl)amino;
R² ausgewählt ist aus der Gruppe, bestehend aus Wasserstoff, C₁₋₄-Alkyl und Trifluormethyl;
R^{2a} ausgewählt ist aus der Gruppe, bestehend aus Wasserstoff, C₁₋₄-Alkyl und Trifluormethyl;
R³ ausgewählt ist aus der Gruppe, bestehend aus Wasserstoff und C₁₋₄-Alkyl;
R⁴ ausgewählt ist aus der Gruppe, bestehend aus Aryl;
wobei die Arylgruppe fakultativ mit einem bis zwei Substituenten substituiert ist, die unabhängig ausgewählt sind aus Halogen, Alkyl, halogensubstituiertem Alkyl, C₁₋₄-Alkylester, Cyano, N(R^{A})₂C(O)-, C₁₋₄-Alkyl-C(O)-NR^{A}-, C₁₋₄-Alkyl-S(O)₀₋₂-, Phenyl-S(O)₀₋₂, C₁₋₄-Alkyl-S(O)₀₋₂-NR^{A}-, Phenyl-S(O)₀₋₂-NR^{A}- und Trifluormethylsulfonyl;
wobei das Phenyl fakultativ substituiert ist mit einem oder mehreren Substituenten, die unabhängig ausgewählt sind aus Halogen, C₁₋₄-Alkyl, C₁₋₄-Alkoxy, Hydroxy, Carboxy, Cyano, Nitro, Amino, C₁₋₄-Alkylamino oder Di(C₁₋₄-alkyl)amino;
R⁵ ausgewählt ist aus der Gruppe, bestehend aus Wasserstoff, C₁₋₄-Alkyl und Trifluormethyl;
oder ein pharmazeutisch annehmbares Salz davon.

3. Verbindung nach Anspruch 2, **dadurch gekennzeichnet, daß**
R¹ ausgewählt ist aus der Gruppe, bestehend aus Aryl; wobei die Arylgruppe fakultativ mit einem Halogen substituiert ist;
R² ausgewählt ist aus der Gruppe, bestehend aus Wasserstoff und C₁₋₄-Alkyl;
R^{2a} ausgewählt ist aus der Gruppe, bestehend aus Wasserstoff und C₁₋₄-Alkyl;
R³ Wasserstoff ist;
R⁴ ausgewählt ist aus der Gruppe, bestehend aus Aryl, wobei die Arylgruppe mit einem bis drei Substituenten substituiert ist, die unabhängig ausgewählt sind aus Halogen, Cyano, Nitro, C₁₋₄-Alkyl, halogensubstituiertem C₁₋₄-Alkyl und Trifluormethylsulfonyl;
R⁵ ausgewählt ist aus der Gruppe, bestehend aus Wasserstoff und C₁₋₄-Alkyl;
oder ein pharmazeutisch annehmbares Salz davon.

4. Verbindung nach Anspruch 3, **dadurch gekennzeichnet, daß**
R¹ ausgewählt ist aus der Gruppe, bestehend aus 3-Fluorphenyl, 4-Fluorphenyl, 3-Chlorphenyl und 4-Chlorphenyl;
R² ausgewählt ist aus der Gruppe, bestehend aus Wasserstoff und Methyl;
R^{2a} ausgewählt ist aus der Gruppe, bestehend aus Wasserstoff und Methyl;
R³ Wasserstoff ist;
R₄ ausgewählt ist aus der Gruppe, bestehend aus 4-Chlorphenyl, 4-Cyanophenyl, 3-Trifluormethyl-4-cyanophenyl, 3-Trifluormethyl-4-nitrophenyl, 3-Trifluormethyl-4-chlorphenyl, 3-Trifluormethyl-4-fluorphenyl, 2,5-Difluor-4-cyanophenyl, 2-Fluor-3-trifluormethylphenyl und 4-Trifluormethylsulfonylphenyl;
R⁵ ausgewählt ist aus der Gruppe, bestehend aus Wasserstoff und Methyl;
oder ein pharmazeutisch annehmbares Salz davon.

5. Verbindung nach Anspruch 4, **dadurch gekennzeichnet, daß**
R¹ ausgewählt ist aus der Gruppe, bestehend aus 4-Fluorphenyl, 3-Chlorphenyl und 4-Chlorphenyl;
R² ausgewählt ist aus der Gruppe, bestehend aus Wasserstoff und Methyl;
R^{2a} ausgewählt ist aus der Gruppe, bestehend aus Wasserstoff und Methyl;
R³ Wasserstoff ist;
R⁴ ausgewählt ist aus der Gruppe, bestehend aus 3-Trifluormethyl-4-cyanophenyl, 3-Trifluormethyl-4-nitrophenyl, 3-Trifluormethyl-4-chlorphenyl und 4-Trifluormethylsulfonylphenyl;
R⁵ ausgewählt ist aus der Gruppe, bestehend aus Wasserstoff und Methyl;
oder ein pharmazeutisch annehmbares Salz davon.

6. Verbindung nach Anspruch 5, **dadurch gekennzeichnet, daß**
R¹ ausgewählt ist aus der Gruppe, bestehend aus 4-Fluorphenyl und 4-Chlorphenyl;
R² Wasserstoff ist;
R^{2a} Wasserstoff ist;
R³ Wasserstoff ist;
R⁴ ausgewählt ist aus der Gruppe, bestehend aus 3-Trifluormethyl-4-nitrophenyl und 3-Trifluormethyl-4-chlorphenyl;
R⁵ ausgewählt ist aus der Gruppe, bestehend aus Wasserstoff und Methyl;
oder ein pharmazeutisch annehmbares Salz davon.

7. Pharmazeutische Zusammensetzung, die einen pharmazeutisch annehmbaren Trägerstoff und eine Verbindung nach Anspruch 1 umfaßt.

8. Pharmazeutische Zusammensetzung, die hergestellt ist durch Vermischen einer Verbindung von Anspruch 1 und eines pharmazeutisch annehmbaren Trägerstoffes.

9. Verfahren zur Herstellung einer pharmazeutischen Zusammensetzung, das das Vermischen einer Verbindung von Anspruch 1 und eines pharmazeutisch annehmbaren Trägerstoffes umfaßt.

10. Verbindung nach einem der Ansprüche 1 bis 6 zur Verwendung in der Medizin.

11. Verwendung der Verbindung nach Anspruch 1 zur Herstellung eines Arzneimittels zur Behandlung einer Störung, die durch einen Androgen-Rezeptor vermittelt ist, bei einem Patienten, der derselben bedarf.

12. Verwendung der Verbindung nach Anspruch 1 zur Herstellung eines Arzneimittels zur Behandlung eines Zustandes, der ausgewählt ist aus der Gruppe, bestehend aus Prostatakarzinom, gutartiger Prostatahyperplasie (BPH), Hirsutismus, Alopezie, Anorexia nervosa, Brustkrebs, Akne, AIDS, Kachexie, männlicher Empfängnisverhütung und männlicher Leistung, bei einem Patienten, der derselben bedarf.

## Revendications

1. Composé de formule (1) 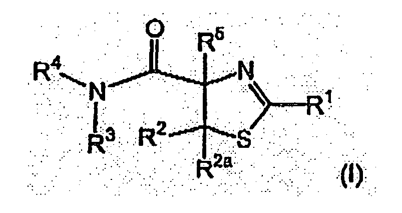 où
R¹ est sélectionné dans le groupe consistant en un aryle et un hétéroaryle ;
où le groupe hétéroaryle est lié par un atome de carbone ;
où le groupe aryle ou hétéroaryle est facultativement substitué par un à quatre substituants indépendamment sélectionnés parmi halogène, alkyle, alkyle substitué en halogène, alkyl ester C₁₋₄, cyano, N(R^{A})₂C(O)-, alkyl C₁₋₄ C(O)-NR^{A}-, alkyl C₁₋₄ S(O)₀₋₂-, phényl S(O)₀₋₂-, alkyl C₁₋₄ S(O)₀₋₂-NR^{A-}, phényl S(O)₀₋₂-NR^{A}- et trifluorométhyl-sulfonyle ;
où le phényle est facultativement substitué par un ou plusieurs substituants indépendamment sélectionnés parmi halogène, alkyle C₁₋₄, alcoxy C₁₋₄, hydroxy, carboxy, cyano, nitro, amino, alkylamino C₁₋₄, ou dialkylamino C₁₋₄ ;
R^{A} est indépendamment sélectionné parmi hydrogène ou alkyle C₁₋₄ ;
R² et R^{2a} sont chacun indépendamment sélectionnés dans le groupe consistant en hydrogène, alkyle C₁₋₄ et alkyle C₁₋₄ substitué en halogène ;
R³ est sélectionné dans le groupe consistant en hydrogène et alkyle C₁₋₄ ;
R⁴ est sélectionné dans le groupe consistant en aryle et hétéroaryle ;
où le groupe hétéroaryle est lié par un atome de carbone ;
où le groupe aryle ou hétéroaryle est facultativement substitué par un à quatre substituants indépendamment sélectionnés parmi halogène, alkyle, alkyle substitué en halogène, alkyl ester C₁₋₄, cyano, N(R^{A})₂C(O)-, alkyl C₁₋₄-C(O)-NR^{A}-, alkyl C₁₋₄-S(O)₀₋₂-, phényl-S(O)₀₋₂, alkyle C₁₋₄-S(O)₀₋₂NR^{A}-, phényl-S(O)₀₋₂-NR^{A} et trifluorométhyl-sulfonyle ;
où le phényle est facultativement substitué par un ou plusieurs substituants indépendamment sélectionnés parmi halogène, alkyle C₁₋₄, alcoxy C₁₋₄, hydroxy, carboxy, cyano, nitro, amino, alkylamino C₁₋₄, ou dialkylamino C₁₋₄ ;
R⁵ est sélectionné dans le groupe consistant en hydrogène, alkyle C₁₋₄ et alkyle C₁₋₄ substitué en halogène ;
ou bien un sel pharmaceutiquement acceptable.

2. Composé selon la revendication 1 où
R¹ est sélectionné dans le groupe consistant en aryle ;
où le groupe aryle est facultativement substitué par un ou deux substituants indépendamment sélectionnés parmi halogène, alkyle, alkyle substitué en halogène, alkyl ester C₁₋₄, cyano, N(R^{A})₂C(O)-, alkyl C₁₋₄-C(O)-NR^{A}-, alkyl C₁₋₄-S(O)₀₋₂-, phényl-S(O)₀₋₂, alkyl C₁₋₄-S(O)₀₋₂-NR^{A}-, phényl-S(O)₀₋₂-NR^{A} et trifluorométhyl-sulfonyle ;
où le phényle est facultativement substitué par un ou plusieurs substituants indépendamment sélectionnés parmi halogène, alkyle C₁₋₄, alcoxy C₁₋₄, hydroxy, carboxy, cyano, nitro, amino, alkylamino C₁₋₄, ou dialkylamino C₁₋₄ ;
R² est sélectionné dans le groupe consistant en hydrogène, alkyle C₁₋₄ et trifluorométhyle ;
R^{2a} est sélectionné dans le groupe consistant en hydrogène, alkyle C₁₋₄ et trifluorométhyle ;
R³ est sélectionné dans le groupe consistant en hydrogène et alkyle C₁₋₄ ;
R⁴ est sélectionné dans le groupe consistant en aryle ;
où le groupe aryle est facultativement substitué par un ou deux substituants indépendamment sélectionnés parmi halogène, alkyle, alkyle substitué en halogène, alkyl ester C₁₋₄, cyano, N(R^{A})₂C(O)-, alkyl C₁₋₄-C(O)-NR^{A}-, alkyl C₁₋₄-S(O)₀₋₂-, Phényl-S(O)₀₋₂, alkyl C₁₋₄-S(O)₀₋₂-NR^{A}-, phényl-S(O)₀₋₂-NR^{A} et trifluorométhyl-sulfonyle ;
où le phényle est facultativement substitué par un ou plusieurs substituants indépendamment sélectionnés parmi halogène, alkyle C₁₋₄, alcoxy C₁₋₄, hydroxy, carboxy, cyano, nitro, amino, alkylamino C₁₋₄, ou dialkylamino C₁₋₄ ;
R⁵ est sélectionné dans le groupe consistant en hydrogène, alkyle C₁₋₄ et trifluorométhyle ;
ou un sel pharmaceutiquement acceptable.

3. Composé selon la revendication 2 où
R¹ est sélectionné dans le groupe consistant en aryle ; où le groupe aryle est facultativement substitué par un halogène ;
R² est sélectionné dans le groupe consistant en hydrogène et alkyle C₁₋₄ ;
R^{2a} est sélectionné dans le groupe consistant en hydrogène et alkyle C₁₋₄ ;
R³ est hydrogène ;
R⁴ est sélectionné dans le groupe consistant en aryle, où le groupe aryle est substitué par un à trois substituants indépendamment sélectionnés parmi halogène, cyano, nitro, alkyle C₁₋₄, alkyle C₁₋₄ substitué en halogène et trifluorométhyl-sulfonyle ;
R⁵ est sélectionné dans le groupe consistant en hydrogène et alkyle C₁₋₄ ;
ou un sel pharmaceutiquement acceptable.

4. Composé selon la revendication 3 où
R¹ est sélectionné dans le groupe consistant en 3-fluorophényle, 4-fluorophényle, 3-chlorophényle et 4-chlorophényle ;
R² est sélectionné dans le groupe consistant en hydrogène et méthyle ;
R^{2a} est sélectionné dans le groupe consistant en hydrogène et méthyle ;
R³ est hydrogène ;
R⁴ est sélectionné dans le groupe consistant en 4-chlorophényle, 4-cyanophényle, 3-trifluorométhyl-4-cyanophényle, 3-trifluorométhyl-4-nitro-phényle, 3-trifluorométhyl-4-chloro-phényle; 3-trifluorométhyl-4-fluoro-phényle, 2,5-difluoro-4-cyano-phényle, 2-fluoro-3-trifluorométhyl-phényle et 4-trifluorométhyl-sulfonyl-phényle ;
R⁵ est sélectionné dans le groupe consistant en hydrogène et méthyle ;
ou un sel pharmaceutiquement acceptable.

5. Composé selon la revendication 4 où
R¹ est sélectionné dans le groupe consistant en 4-fluorophényle, 3-chlorophényle et 4-chlorophényle ;
R² est sélectionné dans le groupe consistant en hydrogène et méthyle ;
R^{2a} est sélectionné dans le groupe consistant en hydrogène et méthyle ;
R³ est hydrogène ;
R⁴ est sélectionné dans le groupe consistant en 3-trifluorométhyl-4-cyano-phényle, 3-trifluorométhyl-4-nitro-phényle, 3-trifluorométhyl-4-chloro-phényle et 4-trifluorométhylsulfonyl-phényle ;
R⁵ est sélectionné dans le groupe consistant en hydrogène et méthyle ;
ou un sel pharmaceutiquement acceptable.

6. Composé selon la revendication 5 où
R¹ est sélectionné dans le groupe consistant en 4-fluorophényle et 4-chlorophényle
R² est hydrogène ;
R^{2a} est hydrogène ;
R³ est hydrogène ;
R⁴ est sélectionné dans le groupe consistant en 3-trifluorométhyl-4-nitrophényle et 3-trifluorométhyl-4-chloro-phényle ;
R⁵ est sélectionné dans le groupe consistant en hydrogène et méthyle ;
ou un sel pharmaceutiquement acceptable.

7. Composition pharmaceutique comprenant un support pharmaceutiquement acceptable et un composé de la revendication 1.

8. Composition pharmaceutique faite en mélangeant un composé de la revendication 1 et un support pharmaceutiquement acceptable.

9. Procédé de production d'une composition pharmaceutique comprenant le mélange d'un composé de la revendication 1 et d'un support pharmaceutiquement acceptable.

10. Composé selon l'une quelconque des revendications 1 à 6 à utiliser en médecine.

11. Utilisation du composé de la revendication 1 dans la fabrication d'un médicament pour le traitement d'un trouble provoqué par un récepteur androgène chez un sujet le nécessitant.

12. Utilisation du composé de la revendication 1 dans la fabrication d'un médicament pour le traitement d'une condition sélectionnée dans le groupe consistant en carcinome de la prostate, hyperplasie bénigne de la prostate (BPH), hirsutisme, alopécie, anorexie nerveuse, cancer du sein, acné, sida, cachexie, contraception masculine et performance masculine chez un sujet le nécessitant.
